# EUROPEAN PATENT APPLICATION

(11) **EP 4 825 352 A1**
(43) Date of publication of application: **21.10.2026**
(21) Application number: 25777618.7
(22) Date of filing: 24.03.2025
(51) Int. Cl.: A61F 13/49, A61F 13/514

(54) **ABSORBENT ARTICLE**

(30) Priority: 25.03.2024 JP 2024048787; 25.03.2024 JP 2024048788; 25.03.2024 JP 2024048789; 31.05.2024 JP 2024089430
(71) Applicant: Unicharm Corporation, Ehime 799-0111 (JP)
(72) Inventor: KATO, Nobuyuki, Kanonji-shi, Kagawa 769-1602 (JP); SUZUKI, Kenta, Kanonji-shi, Kagawa 769-1602 (JP)
(74) Representative: Staeger & Sperling Partnerschaftsgesellschaft mbB
(86) International application number: PCT/JP2025/011474
(87) International publication number: WO 2025/205617

(57) **Abstract**

An absorbent article (1) is provided with a crotch elastic region (ER) in a crotch part thereof, and has a peripheral region (AR) surrounding the crotch elastic region (ER). When the absorbent article (1) is viewed from the non-skin side, the absorbent article (1) has a background color region (61) and a specific color region (60) having a certain specific color different from the background color. When the ratio obtained by dividing the total area of the specific color region (60) provided in the crotch elastic region (ER) by the area of the crotch elastic region (ER) is defined as a first area ratio, and the ratio obtained by dividing the total area of the specific color region (60) provided in the peripheral region (AR) by the area of the peripheral region (AR) is defined as a second area ratio, the absolute value of the difference between the first area ratio and the second area ratio is equal to or greater than the first area ratio or equal to or greater than the second area ratio.

## Description

### FIELD

The present invention relates to an absorbent article.

### BACKGROUND

A disposable diaper disclosed in Patent Literature 1 includes a first buttocks elastic member that extends in the longitudinal direction and is located on a non-skin-contacting-surface side with respect to an absorbent core. Due to contraction of the first buttocks elastic member, upward tension acts on the diaper, thereby reducing a gap between the diaper and a crotch portion of a wearer. This improves performance of preventing leakage of excrement and also enhances a sense of security during wear due to an appropriate fit.

### [CITATION LIST]

### [PATENT LITERATURE]

Patent Literature 1: Japanese Patent Application Publication No. 2010-131196

### SUMMARY

### [TECHNICAL PROBLEM]

However, merely providing an elastic member on the buttocks as in Patent Literature 1 may fail to make a user notice the presence of the elastic member on the buttocks. In this case, although the elastic member improves close contact with the wearer's crotch portion, the user may fail to recognize this benefit and continue to feel anxiety about leakage during use. Furthermore, users tend to desire a product in which the absorbent core is less likely to sag, based on user's experiences in which, when the absorbent core is pinched by forces from the inner sides of thighs and sags, or when the absorbent core sags due to its weight after absorbing excreted liquid, a gap is formed in the crotch portion, leakage is likely to occur, and wearing comfort is reduced. Therefore, users can use the absorbent article with peace of mind by recognizing that, before use, the absorbent core is less likely to sag due to the elastic member.

The present invention has been made in light of conventional problems such as that described above, and an aspect of the present invention is to provide an absorbent article that makes a user more likely to recognize, before use, that the absorbent article has good close contact with a crotch portion of a wearer and that an absorbent core is less likely to sag.

### [SOLUTION TO PROBLEM]

A main aspect of the present invention for achieving the above-described aspect is an absorbent article having, in an unfolded and stretched state, a longitudinal direction, a lateral direction, and a thickness direction that intersect one another, the absorbent article including: an absorbent core; a crotch elastic region delimited by a stretchable portion of a crotch elastic member; and a peripheral region surrounding the crotch elastic region, the crotch elastic member being provided on a non-skin side with respect to the absorbent core, the crotch elastic member stretching and contracting along the longitudinal direction, at least a part of the crotch elastic region being provided in a crotch portion corresponding to a central region, the central region being obtained by dividing the absorbent article into three equal regions in the longitudinal direction, the peripheral region being a region that, in the lateral direction, extends from a position spaced apart leftward from a left end of the crotch elastic region by a length equal to half a width of the crotch elastic region, to a position spaced apart rightward from a right end of the crotch elastic region by the length equal to half the width of the crotch elastic region, and that, in the longitudinal direction, extends from a position spaced apart toward one side from a one-side end of the crotch elastic region by a length equal to the width of the crotch elastic region, to a position spaced apart toward another side from an other-side end of the crotch elastic region by the length equal to the width of the crotch elastic region, when the absorbent article is viewed from the non-skin side, the absorbent article including: a background-color region having a background color; and a specific-color region having a specific color different from the background color, the background-color region being a region that occupies the largest area in the crotch portion, a first area ratio being a ratio obtained by dividing a total area of the specific-color region provided in the crotch elastic region by an area of the crotch elastic region, a second area ratio being a ratio obtained by dividing a total area of the specific-color region provided in the peripheral region by an area of the peripheral region, an absolute value of a difference between the first area ratio and the second area ratio being equal to or greater than the first area ratio, or being equal to or greater than the second area ratio. Features of the present invention other than the above will become clear by reading the description of the present specification with reference to the accompanying drawings.

### [ADVANTAGEOUS EFFECT OF THE INVENTION]

According to the present invention, it is possible to provide an absorbent article that makes a user more likely to recognize, before use, that the absorbent article has good close contact with a crotch portion of a wearer and that an absorbent core is less likely to sag.

### [Brief Description of the Drawings]

FIG. 1 is a schematic front view of an underpants-shaped disposable diaper 1.
FIG. 2 is a schematic plan view of the diaper 1 in an unfolded and stretched state as viewed from a skin side.
FIG. 3 is a schematic cross-sectional view taken along the C-C line in FIG. 2.
FIG. 4 is a schematic cross-sectional view of an absorbent main body 10.
FIG. 5 is a view illustrating the arrangement of crotch elastic members 40.
FIG. 6 is a view illustrating the arrangement of the crotch elastic members 40 according to a modified example.
FIG. 7 is a view illustrating the arrangement of crotch elastic members 40 according to a modified example.
FIG. 8 is an illustrative view of specific-color regions 60.
FIG. 9 is an enlarged view of a crotch elastic region ER and a peripheral region AR.
FIGS. 10A and 10B are illustrative views of a specific-color region 60 according to a modified example.
FIGS. 11A and 11B are illustrative views of specific-color regions 60 according to a modified example.
FIG. 12 is an illustrative view of specific-color regions 60 according to a modified example.
FIG. 13A is a partial cross-sectional view of an absorbent main body 10, and FIG. 13B is an illustrative view of joining portions 70 between a back sheet 13 and an elastic-member cover sheet 17.
FIG. 14 is an illustrative view of a specific-color region 60 of the diaper 1 according to a second embodiment.

### [DESCRIPTION OF THE EMBODIMENTS]

At least the following matters will become clear with description of this specification and attached drawings. Aspect 1 is an absorbent article having, in an unfolded and stretched state, a longitudinal direction, a lateral direction, and a thickness direction that intersect one another, the absorbent article including: an absorbent core; a crotch elastic region delimited by a stretchable portion of a crotch elastic member; and a peripheral region surrounding the crotch elastic region, the crotch elastic member being provided on a non-skin side with respect to the absorbent core, the crotch elastic member stretching and contracting along the longitudinal direction, at least a part of the crotch elastic region being provided in a crotch portion corresponding to a central region, the central region being obtained by dividing the absorbent article into three equal regions in the longitudinal direction, the peripheral region being a region that, in the lateral direction, extends from a position spaced apart leftward from a left end of the crotch elastic region by a length equal to half a width of the crotch elastic region, to a position spaced apart rightward from a right end of the crotch elastic region by the length equal to half the width of the crotch elastic region, and that, in the longitudinal direction, extends from a position spaced apart toward one side from a one-side end of the crotch elastic region by a length equal to the width of the crotch elastic region, to a position spaced apart toward another side from an other-side end of the crotch elastic region by the length equal to the width of the crotch elastic region, when the absorbent article is viewed from the non-skin side, the absorbent article including: a background-color region having a background color; and a specific-color region having a specific color different from the background color, the background-color region being a region that occupies the largest area in the crotch portion, a first area ratio being a ratio obtained by dividing a total area of the specific-color region provided in the crotch elastic region by an area of the crotch elastic region, a second area ratio being a ratio obtained by dividing a total area of the specific-color region provided in the peripheral region by an area of the peripheral region, an absolute value of a difference between the first area ratio and the second area ratio being equal to or greater than the first area ratio, or being equal to or greater than the second area ratio.

According to Aspect 1, the difference between the area ratio of the specific-color region in the crotch elastic region and the area ratio of the specific-color region in the peripheral region becomes greater, and the crotch elastic region stands out. As a result, the user is more likely to notice the presence of the crotch elastic members before use of the absorbent article, and can recognize that the absorbent article is a product that has good close contact with the wearer's crotch portion and is unlikely to sag. Thus, the concern about leakage and an impression that the absorbent article is uncomfortable to wear can be eliminated. This allows the user to use the absorbent article with peace of mind.

### Aspect 2

The absorbent article according to Aspect 1, wherein in an unfolded and stretched state, the specific-color region in which a longitudinal length is longer than a lateral length is provided in at least one of the crotch elastic region and the peripheral region.

According to Aspect 2, the user who looks at the specific-color region that is long in the longitudinal direction is more likely to get the impression that the crotch elastic member stretches and contracts in the longitudinal direction. Therefore, the user is more likely to recognize that the absorbent article is a product in which the crotch elastic member maintains a state in which the absorbent core is pushed up toward the skin side, and that the absorbent article has good close contact with the wearer's crotch portion and is unlikely to sag.

### Aspect 3

The absorbent article according to Aspect 2, wherein in an unfolded and stretched state, in the crotch elastic region, a plurality of the specific-color regions are arranged in the lateral direction, the plurality of specific-color regions each having a strip shape and extending along the longitudinal direction.

According to Aspect 3, the specific-color region becomes an elongated region, and the user who looks at the specific-color region is even more likely to get the impression that the crotch elastic member stretches and contracts in the longitudinal direction. Therefore, the user is more likely to recognize that the absorbent article is a product that has good close contact with the wearer's crotch portion and is unlikely to sag.

### Aspect 4

The absorbent article according to any one of Aspects 1 to 3, wherein in an unfolded and stretched state, in at least one of the longitudinal direction and the lateral direction, a total length of the specific-color region provided in one of the crotch elastic region and the peripheral region is equal to or greater than half the length of the one of the crotch elastic region and the peripheral region.

According to Aspect 4, in at least one of the longitudinal direction and the lateral direction, the specific-color region is provided over a wide range of one of the crotch elastic region and the peripheral region. As a result, the crotch elastic region stands out, and the user is likely to notice the presence of the crotch elastic member.

### Aspect 5

The absorbent article according to any one of Aspects 1 to 4, wherein the first area ratio is equal to or greater than 1/2, or the second area ratio is equal to or greater than 1/2.

According to Aspect 5, compared to the case where the first area ratio is less than 1/2 or the case where the second area ratio is less than 1/2, the crotch elastic region stands out, making the user more likely to notice the presence of the crotch elastic member.

### Aspect 6

The absorbent article according to any one of Aspects 1 to 5, wherein the specific-color region is not provided in the peripheral region.

According to Aspect 6, since the user focuses attention directly on the crotch elastic region, the user is more likely to notice the presence of the crotch elastic member due to wrinkles caused by contraction of the crotch elastic member.

### Aspect 7

The absorbent article according to any one of Aspects 1 to 6, wherein in an unfolded and stretched state, in the crotch elastic region, a plurality of the crotch elastic members each having a string-like shape are arranged in the lateral direction, and a stretchable portion of the crotch elastic member has a portion that overlaps the specific-color region in the thickness direction.

According to Aspect 7, since the user focuses attention directly on the stretchable portion of the crotch elastic member, the user is more likely to notice the presence of the crotch elastic member due to wrinkles caused by contraction of the crotch elastic member.

### Aspect 8

The absorbent article according to any one of Aspects 1 to 7, wherein in an unfolded and stretched state, in the crotch elastic region, a plurality of the crotch elastic members each having a string-like shape are arranged in the lateral direction, and in a region outside the crotch elastic region in the longitudinal direction, a non-stretchable portion of the crotch elastic member has a portion that does not overlap the specific-color region in the thickness direction.

According to Aspect 8, it is possible to prevent the user from mistakenly recognizing that the non-stretchable portion of the crotch elastic member stretches and contracts, thereby enabling the user to correctly identify the position where actual stretching and contraction occur. Furthermore, the non-stretchable portion is unlikely to stand out, in the crotch elastic member that freely stretches and contracts. As a result, it is possible to suppress reduction in the impression that the crotch elastic member stretches and contracts in the longitudinal direction.

In addition, deterioration in the appearance (aesthetic quality) of the absorbent article can also be suppressed.

### Aspect 9

The absorbent article according to any one of Aspects 1 to 8, wherein the absorbent article includes: a liquid-impermeable back sheet; and a non-skin-side sheet provided on the non-skin side with respect to the back sheet, the crotch elastic member is provided between the back sheet and the non-skin-side sheet, the specific-color region is formed by coloring the back sheet, and in an unfolded and stretched state, in the crotch elastic region, a plurality of the crotch elastic members each having a string-like shape are arranged in the lateral direction, and a joining portion that joins the back sheet and the non-skin-side sheet is provided between the plurality of crotch elastic members arranged in the lateral direction.

According to Aspect 9, lifting of the non-skin-side sheet relative to the back sheet can be suppressed, thereby enhancing the visibility of the specific-color region formed in the back sheet. As a result, the crotch elastic region stands out, making the user likely to recognize the presence of the crotch elastic member.

### Aspect 10

The absorbent article according to any one of Aspects 1 to 9, wherein the absorbent article includes: a liquid-impermeable back sheet; and a non-skin-side sheet provided on the non-skin side with respect to the back sheet, the crotch elastic member is provided between the back sheet and the non-skin-side sheet, in an unfolded and stretched state, in the crotch elastic region, a plurality of the crotch elastic members each having a string-like shape are arranged in the lateral direction, a joining portion that joins the back sheet and the non-skin-side sheet is provided between the plurality of crotch elastic members arranged in the lateral direction, and the joining portion extends outward beyond the crotch elastic region in the longitudinal direction.

According to Aspect 10, the joining portion restricts bending of the non-stretchable portion of the crotch elastic member in the lateral direction. As a result, it is possible to suppress reduction in the impression that the crotch elastic member stretches and contracts in the longitudinal direction. In addition, deterioration in the appearance (aesthetic quality) of the absorbent article can also be suppressed.

### Aspect 11

The absorbent article according to any one of Aspects 1 to 10, wherein the specific-color region is formed by coloring the crotch elastic member.

According to Aspect 11, the user is more likely to focus attention directly on the crotch elastic member, and is more likely to notice the presence of the crotch elastic member. In addition, the user can correctly identify the position where actual stretching and contraction occur.

### Aspect 12

The absorbent article according to any one of Aspects 1 to 11, wherein in an unfolded and stretched state, the absorbent article includes a waist elastic region that stretches and contracts along the lateral direction at least at a longitudinal one-end portion of the absorbent article, and when the absorbent article is viewed from the non-skin side, the waist elastic region includes a waist colored region colored along the lateral direction.

According to Aspect 12, the waist colored region stands out, and the user is more likely to notice the presence of the waist elastic region. Thus, the user can recognize that the absorbent article is a product that has good close contact with the wearer's waist portion, thereby alleviating the concern about leakage and eliminating the impression that the absorbent article is uncomfortable to wear. This allows the user to use the absorbent article with peace of mind.

### Aspect 13

The absorbent article according to any one of Aspects 1 to 12, wherein in an unfolded and stretched state, the absorbent article includes side-portion elastic regions provided in two lateral side portions of an absorbent main body including the absorbent core, respectively, the side-portion elastic regions stretching and contracting along the longitudinal direction, and when the absorbent article is viewed from the non-skin side or a skin side, each of the side-portion elastic regions includes a side-portion colored region colored along the longitudinal direction.

According to Aspect 13, the side-portion colored region stands out, and the user is more likely to notice the presence of the side-portion elastic region. As a result, the user can recognize that the absorbent article is a product that has good fit around the leg portions of the wearer, thereby alleviating the concern about leakage and eliminating the impression that the absorbent article is uncomfortable to wear. This allows the user to use the absorbent article with peace of mind.

### Aspect 14

The absorbent article according to Aspect 12, wherein in an unfolded and stretched state, the absorbent article includes side-portion elastic regions provided in two lateral side portions of an absorbent main body including the absorbent core, respectively, the side-portion elastic regions stretching and contracting along the longitudinal direction, when the absorbent article is viewed from the non-skin side or a skin side, each of the side-portion elastic regions includes a side-portion colored region colored along the longitudinal direction, and the specific-color region in the crotch elastic region as viewed from the non-skin side, the waist colored region as viewed from the non-skin side, and each of the side-portion colored regions as viewed from the non-skin side or the skin side are similar in color.

According to Aspect 14, a sense of visual unity is created among the three elastic regions (the crotch elastic region, the waist elastic region, and the side-portion elastic region). Thus, the user is more likely to visually and intuitively recognize that the crotch portion, the waist portion, and the leg portions of the absorbent article each stretch and contract. In addition, the appearance (aesthetic quality) of the absorbent article is also improved. Furthermore, due to the three elastic regions that are similar in color, the user is more likely to recognize that the absorbent article fits all around the wearer (particularly that the fit is good at regions where leakage is likely to occur), thereby allowing the user to use the absorbent article with peace of mind.

### Aspect 15

The absorbent article according to Aspect 12 or 14, wherein in an unfolded and stretched state, the absorbent article includes side-portion elastic regions provided in two lateral side portions of an absorbent main body including the absorbent core, respectively, the side-portion elastic regions stretching and contracting along the longitudinal direction, when the absorbent article is viewed from the non-skin side or a skin side, each of the side-portion elastic regions includes a side-portion colored region colored along the longitudinal direction, and a maximum lateral width of the specific-color region in the crotch elastic region is greater than a maximum longitudinal width of the waist colored region and a maximum lateral width of each of the side-portion colored regions.

According to Aspect 15, the crotch elastic region stands out the most, making the user likely to notice the presence of the crotch elastic member. Therefore, the user can recognize that the absorbent article is a product that has good close contact with the wearer's crotch portion and is unlikely to sag. This allows the user to use the absorbent article with peace of mind.

### Aspect 16

An absorbent article having, in an unfolded and stretched state, a longitudinal direction, a lateral direction, and a thickness direction that intersect one another, the absorbent article including: an absorbent core; a crotch elastic region delimited by a stretchable portion of a crotch elastic member; and a peripheral region surrounding the crotch elastic region, the crotch elastic member being provided on a non-skin side with respect to the absorbent core, the crotch elastic member stretching and contracting along the longitudinal direction, at least a part of the crotch elastic region being provided in a crotch portion corresponding to a central region, the central region being obtained by dividing the absorbent article into three equal regions in the longitudinal direction, the peripheral region being a region that, in the lateral direction, extends from a position spaced apart leftward from a left end of the crotch elastic region by a length equal to half a width of the crotch elastic region, to a position spaced apart rightward from a right end of the crotch elastic region by the length equal to half the width of the crotch elastic region, and that, in the longitudinal direction, extends from a position spaced apart toward one side from a one-side end of the crotch elastic region by a length equal to the width of the crotch elastic region, to a position spaced apart toward another side from an other-side end of the crotch elastic region by the length equal to the width of the crotch elastic region, when the absorbent article is viewed from the non-skin side, the absorbent article including: a background-color region having a background color; and a specific-color region having a specific color different from the background color, the background-color region being a region that occupies the largest area in the crotch portion, in at least one of the longitudinal direction and the lateral direction, a total length of the specific-color region provided in one of the crotch elastic region and the peripheral region being equal to or greater than half a length of the one of the crotch elastic region and the peripheral region.

According to Aspect 16, in at least one of the longitudinal direction and the lateral direction, the specific-color region is provided over a wide range of one of these regions, and the crotch elastic region stands out. As a result, before using the absorbent article, the user is more likely to notice the presence of the crotch elastic member, and can recognize that the absorbent article is a product that has good close contact with the wearer's crotch portion and is unlikely to sag.

### Embodiments

Hereinafter, an underpants-shaped absorbent article according to an embodiment of the present invention will be described with an underpants-shaped disposable diaper for adults (hereinafter also referred to as a "diaper 1") as an example. However, the absorbent article according to the present invention is not limited thereto, and is also applicable to tape-type disposable diapers, sanitary panties, and the like. A wearer of the absorbent article may be an infant, an animal, or other living creatures.

### First Embodiment

### Basic Configuration of Underpants-shaped Disposable Diaper 1

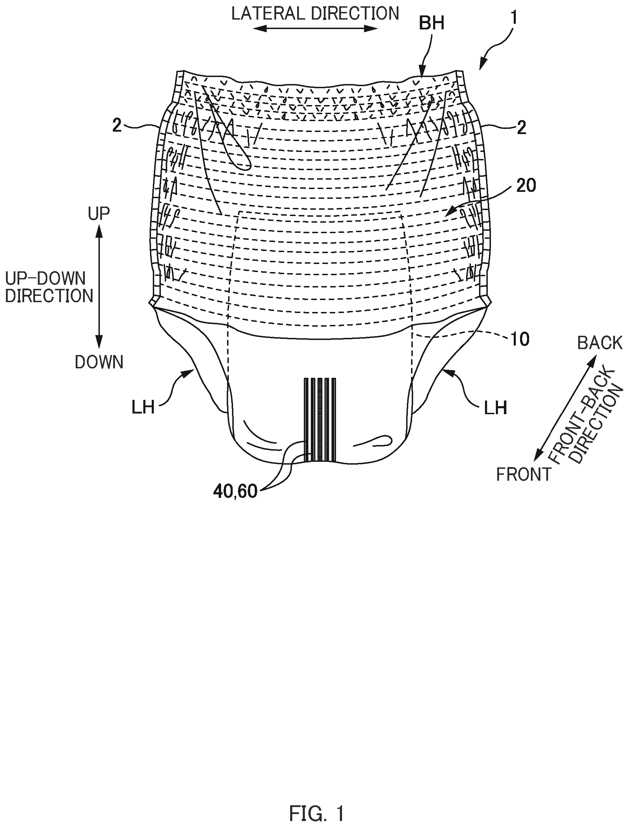

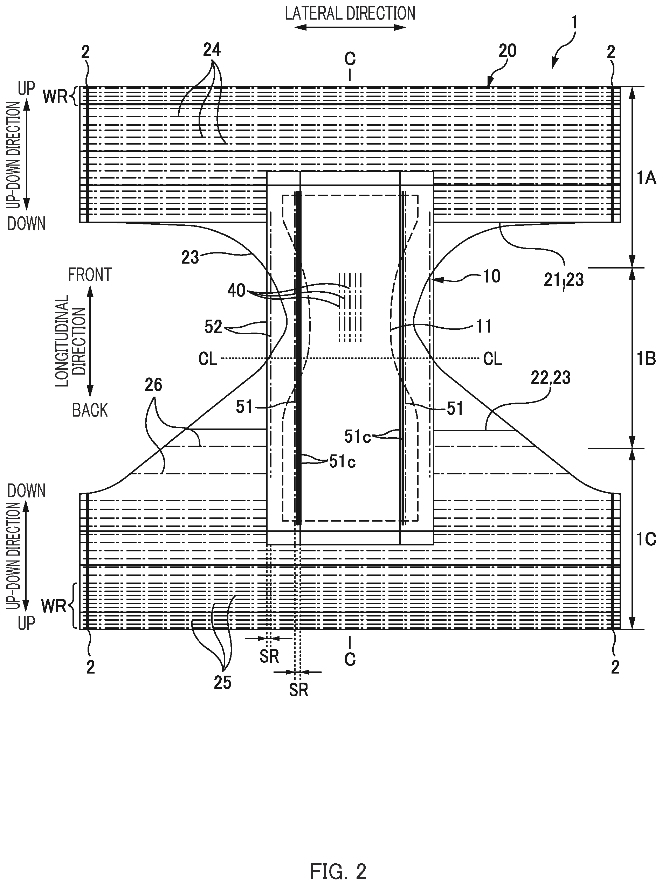

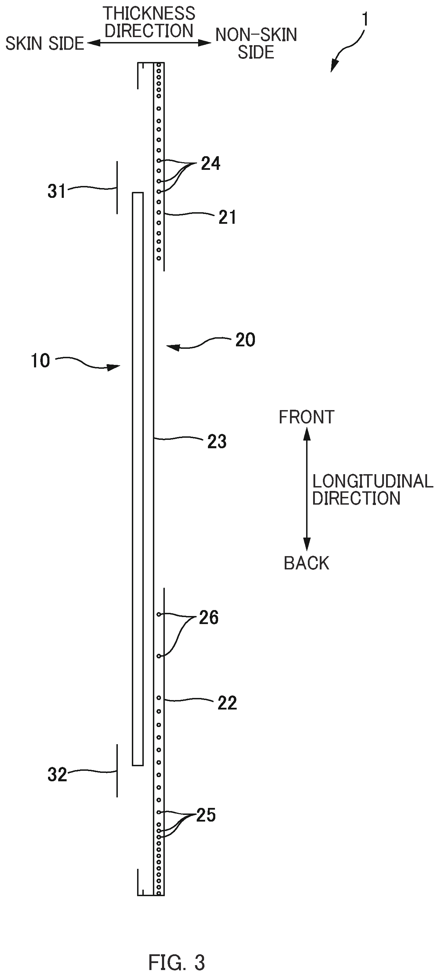

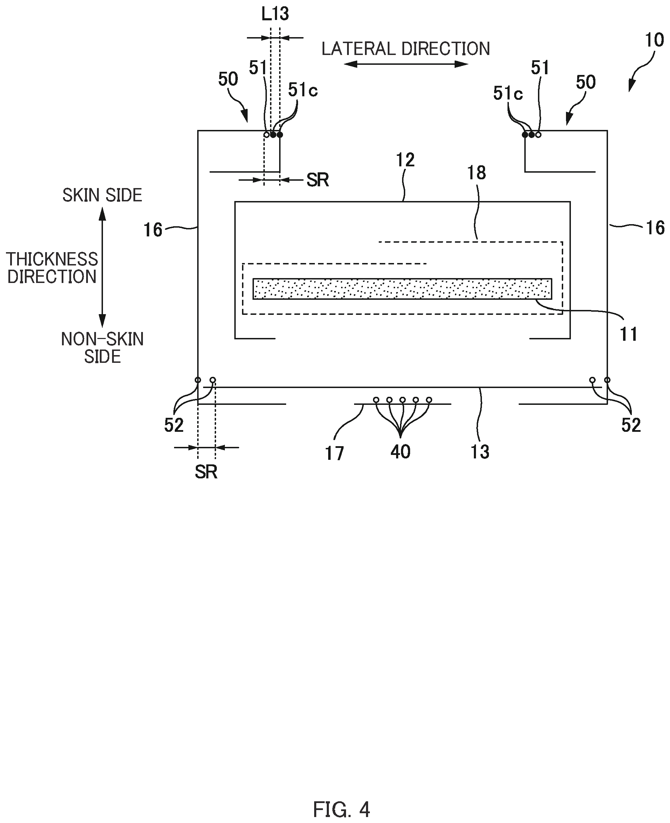

FIG. 1 is a schematic front view of the underpants-shaped disposable diaper 1. FIG. 2 is a schematic plan view of the diaper 1 in an unfolded and stretched state as viewed from a skin side. FIG. 3 is a schematic cross-sectional view taken along the C-C line in FIG. 2. FIG. 4 is a schematic cross-sectional view of an absorbent main body 10. Here, the "unfolded state" of the diaper 1 refers to a state in which the diaper 1 is unfolded in the longitudinal direction and the diaper 1 is spread out flat by unjoining waist joining portions 2, which will be described later.

In the underpants-shaped state shown in FIG. 1, the diaper 1 has an up-down direction, a lateral direction, and a front-back direction that intersect one another, and includes a waist opening BH and a pair of leg openings LH. Also, in the unfolded state shown in FIG. 2, the diaper 1 has a longitudinal direction, a lateral direction, and a thickness direction that intersect one another. The longitudinal direction is a direction extending along the up-down direction of the diaper 1 in the underpants-shaped state. The thickness direction is a direction in which members constituting the diaper 1 are overlaid on each other (see FIG. 4). In the up-down direction, the waist opening BH side is the upper side, and the crotch side is the lower side. In the front-back direction, the abdominal side of the wearer is the front side, and the back side of the wearer is the back side. In the thickness direction, the side that comes into contact with the wearer's body (skin) is the skin side, and the side opposite thereto is the non-skin side.

The "stretched state" of the diaper 1 refers to a state in which, by stretching elastic members (e.g., waist elastic members 24 and 25 and leak-proof-wall elastic members 51) of the diaper 1, the entire diaper 1 is stretched to eliminate wrinkles, or more specifically, is stretched such that the dimensions of constituent members of the diaper 1 (e.g., a top sheet 12 and inner-layer sheet 23, both described later) match or are close to the dimensions of respective members on their own.

As shown in FIG. 3, the diaper 1 includes an absorbent main body 10 and an exterior member 20 located on the non-skin side with respect to the absorbent main body 10. The absorbent main body 10 and the exterior member 20 are joined together using an adhesive (not shown). Cover sheets 31 and 32 that cover two longitudinal end portions of the absorbent main body 10 from the skin side may be provided. This can suppress biting of the edge of the absorbent main body 10 into the wearer and opening of the absorbent main body 10.

In the present specification, as shown in FIG. 2, in the unfolded and stretched state of the diaper 1, when the diaper 1 is divided into three equal regions in the longitudinal direction, the front-side region is referred to as a "front-side portion 1A", the central region is referred to as a "crotch portion 1B", and the back-side region is referred to as a "back-side portion 1C". The diaper 1 in the unfolded state is folded one time at a longitudinal central position CL, and two lateral side portions of the exterior member 20 in the front-side portion 1A and two lateral side portions of the exterior member 20 in the back-side portion 1C are joined together, thereby forming the diaper 1 in the underpants-shaped state shown in FIG. 1. Examples of the joining method for the waist joining portion 2 may include welding or joining using an adhesive.

As shown in FIG. 4, the absorbent main body 10 includes: an absorbent core 11 that absorbs excreted fluid; a liquid-permeable top sheet 12 (for example, a nonwoven fabric sheet) that is arranged on the skin side in the thickness direction with respect to the absorbent core 11; a liquid-impermeable back sheet 13 (for example, a resin film such as polyethylene or polypropylene) that is arranged on the non-skin side in the thickness direction with respect to the absorbent core 11; a pair of side sheets 16; and an elastic-member cover sheet 17. The elastic-member cover sheet 17 is a sheet member that covers, from the non-skin side, crotch elastic members 40 that stretch and contract in the longitudinal direction (details thereof will be described later).

The absorbent core 11 absorbs and retains excreted fluid such as urine. The surface of the absorbent core 11 may be covered with a liquid-permeable core-wrapping sheet 18 (such as tissue paper). Examples of the absorbent core 11 include an absorbent core obtained by shaping liquid-absorbent fibers such as pulp containing superabsorbent polymer (SAP) into a predetermined shape. The absorbent core 11 of the present embodiment has a shape in which the longitudinal central portion is constricted inward in the lateral direction; however, the planar shape of the absorbent core 11 is not particularly limited.

A pair of leak-proof-wall portions 50 extending in the longitudinal direction is provided in two lateral side portions of the absorbent main body 10. As shown in FIG. 4, in the side sheets 16, portions extending from two lateral side portions of the non-skin-side surface of the back sheet 13 are folded at a plurality of locations and are provided at two lateral side portions on the skin-side surface of the top sheet 12, thereby forming the leak-proof-wall portions 50. Leak-proof-wall elastic members 51 (for example, string-like elastic members) in a state of being stretched along the longitudinal direction are attached to the leak-proof-wall portions 50. Due to stretchability of the leak-proof-wall elastic members 51, the leak-proof-wall portions 50 rise toward the wearer's skin side and stand upright, thereby blocking excrement. Outside the leak-proof-wall portions 50 in the lateral direction, leg elastic members 52 are provided along the longitudinal direction. Due to stretchability of the leg elastic members 52, the diaper 1 can fit around the wearer's legs.

The exterior member 20 is a sheet member having a shape in which the longitudinal central portion is constricted inward in the lateral direction. As shown in FIG. 3, the exterior member 20 includes: a front-side outer-layer sheet 21; a back-side outer-layer sheet 22; an inner-layer sheet 23; waist elastic members 24 and 25; and buttocks elastic members 26.

Examples of the front-side outer-layer sheet 21, the back-side outer-layer sheet 22, and the inner-layer sheet 23 may include flexible sheet members such as nonwoven fabric sheets. The inner-layer sheet 23 is a sheet member that forms the outer shape of the exterior member 20 and extends continuously from the front-side portion 1A to the back-side portion 1C via the crotch portion 1B. The front-side outer-layer sheet 21 is arranged on the non-skin side with respect to the inner-layer sheet 23 and on a part of the front-side portion 1A. The front-side outer-layer sheet 21 overlaps the waist joining portion 2 in the longitudinal direction, and the longitudinal length of the front-side outer-layer sheet 21 is the same as that of the waist joining portion 2. The back-side outer-layer sheet 22 is arranged on the non-skin side with respect to the inner-layer sheet 23, and on a part of the crotch portion 1B and the back-side portion 1C. The back-side outer-layer sheet 22 overlaps the waist joining portion 2 in the longitudinal direction and extends toward the crotch side with respect to the waist joining portion 2. In the diaper 1 in the unfolded state, the front-side outer-layer sheet 21 and the back-side outer-layer sheet 22 are spaced apart in the longitudinal direction, and the inner-layer sheet 23 serving as the non-skin-side surface of the diaper 1 is exposed to the outside at a position between the front-side outer-layer sheet 21 and the back-side outer-layer sheet 22.

The waist elastic members 24 and 25 and the buttocks elastic members 26 are elastic members that stretch and contract in the lateral direction, and examples thereof may include string-like elastic members. As shown in FIG. 2, the waist elastic members 24 and 25 overlap the waist joining portion 2 in the longitudinal direction. In the following description, the waist elastic members located in the front-side portion 1A are also referred to as front-side waist elastic members 24, and the waist elastic members located in the back-side portion 1C are also referred to as back-side waist elastic members 25. The buttocks elastic members 26 are located on the crotch side in the longitudinal direction (on the lower side in the up-down direction) with respect to the waist joining portion 2. In the present embodiment, there are two buttocks elastic members 26, but this number is not particularly limited.

In addition, the waist elastic members 24 and 25 and the buttocks elastic members 26 are arranged side by side in the up-down direction in the underpants-shaped state at a position between the inner-layer sheet 23 and the front-side outer-layer sheet 21 or the back-side outer-layer sheet 22, and are fixed while being stretched along the lateral direction. Therefore, the exterior member 20 fits the wearer's waist and buttocks.

The basic configuration of the diaper 1 has been described above, but the configuration of the diaper 1 is merely an example and is not limited thereto. For example, a liquid-permeable intermediate sheet (e.g., a nonwoven fabric sheet) may be arranged between the top sheet 12 and the absorbent core 11. In addition, the exterior member 20 of the present embodiment is constituted by a single member continuously extending in the longitudinal direction from the front side to the back side, but is not limited thereto. For example, the front-side and back-side exterior members may be separate members, and the non-skin-side surface of the absorbent main body 10 may be exposed at the crotch portion, or an exterior member for the crotch portion connecting the separate front-side and back-side exterior members may be provided.

### Crotch Elastic Members 40

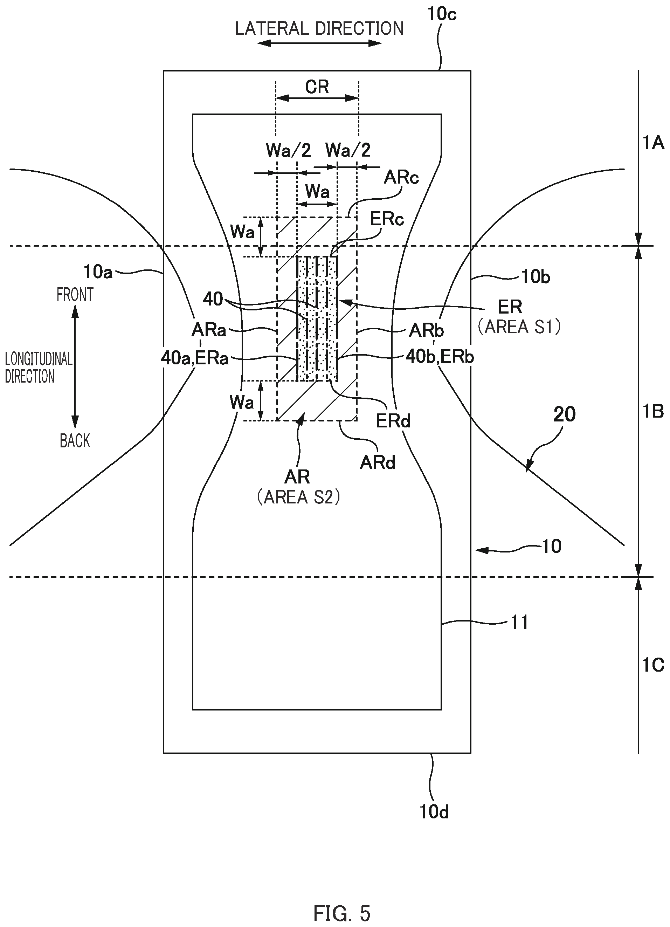

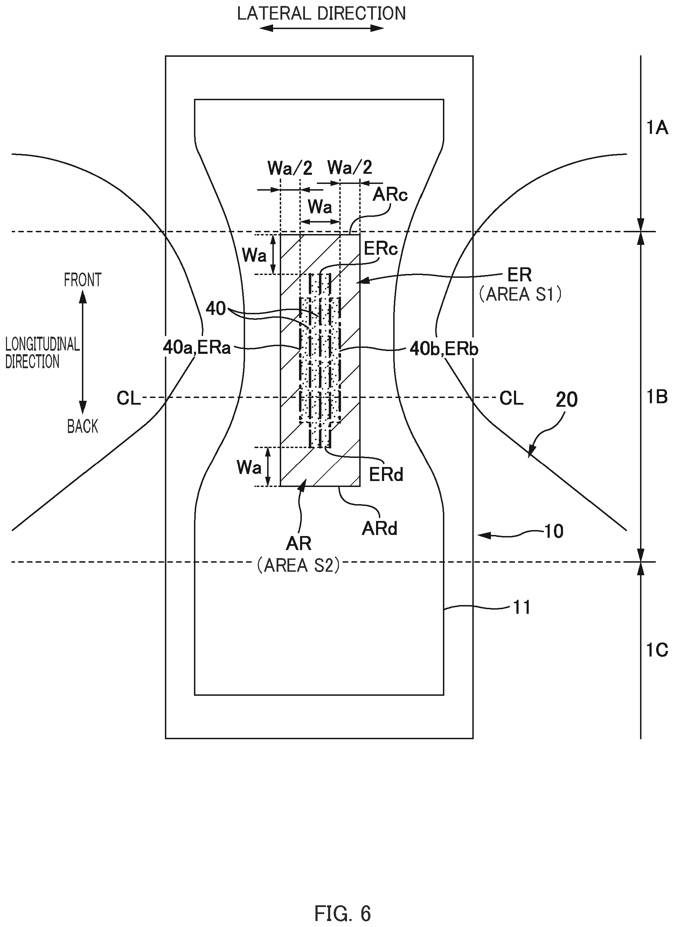

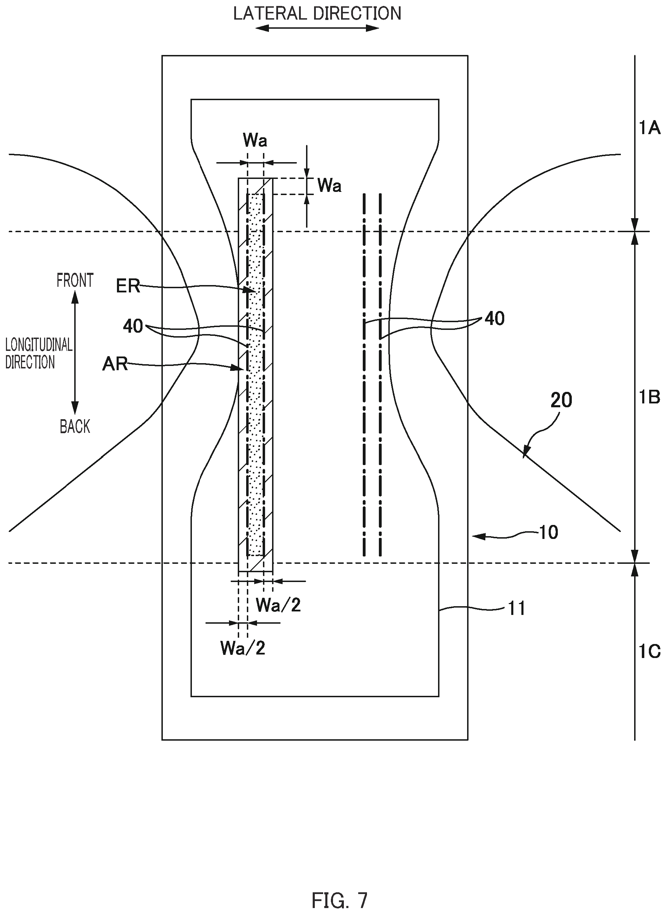

FIG. 5 is a view illustrating the arrangement of the crotch elastic members 40. FIG. 6 and FIG. 7 are views illustrating the arrangement of crotch elastic members 40 according to a modified example. FIGS. 5 to 7 illustrate a part of a schematic plan view of the diaper 1 in an unfolded and stretched state.

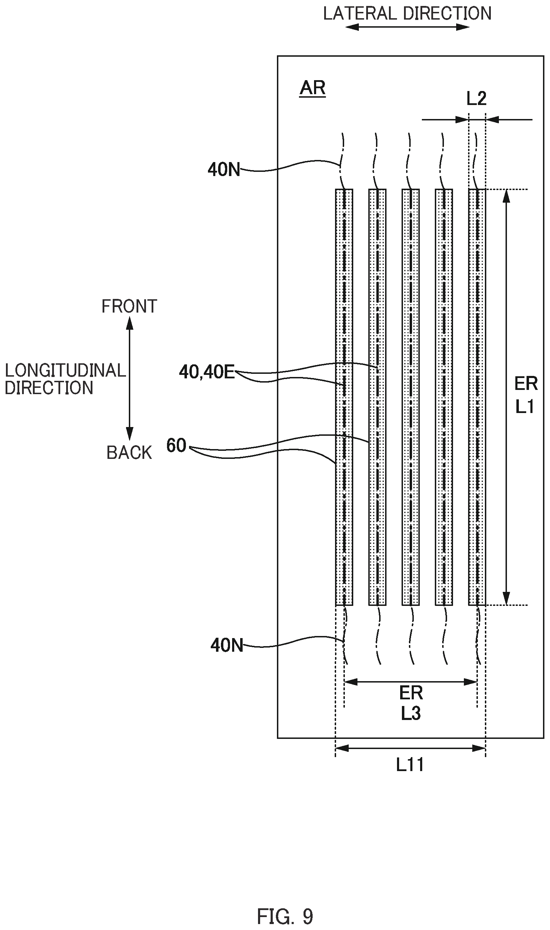

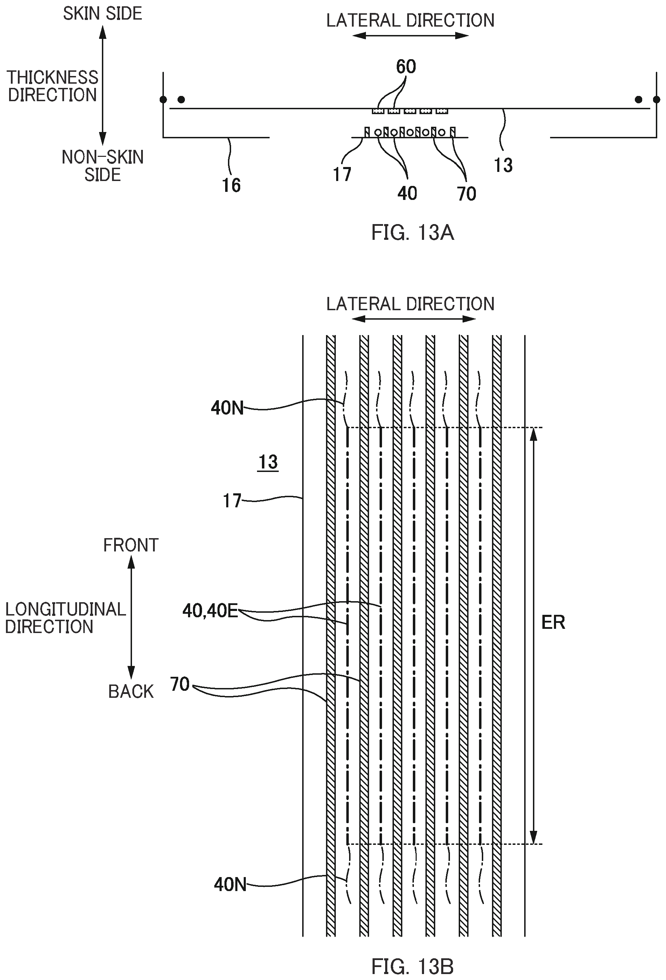

The diaper 1 includes the "crotch elastic members 40" that are provided on the non-skin side with respect to the absorbent core 11 and stretch and contract along the longitudinal direction. The diaper 1 of the present embodiment includes a plurality of string-like elastic members serving as the crotch elastic members 40. As shown in FIG. 4, the plurality of crotch elastic members 40 (five string-like elastic members) are arranged side by side at intervals in the lateral direction at a position between the back sheet 13 and the elastic-member cover sheet 17. Each of the crotch elastic members 40 is attached while being stretched along the longitudinal direction. In the drawings except FIGS. 9 and 13B described below, only a stretchable portion of the crotch elastic members 40 is shown.

A region delimited by the stretchable portion of the crotch elastic members 40 is referred to as a "crotch elastic region ER". At least a part of the crotch elastic region ER is provided in the "crotch portion 1B" that is a central region obtained by dividing the diaper 1 in the unfolded and stretched state into three equal regions in the longitudinal direction. The crotch elastic region ER may be contained within the crotch portion 1B (FIGS. 5 and 6), may extend to the front-side portion 1A (FIG. 7), or may extend to the back-side portion 1C. In addition, the crotch elastic region ER may be arranged so as to span the longitudinal central position CL of the diaper 1, or may be located so as to be biased toward the front side or back side with respect to the longitudinal central position CL. The plurality of crotch elastic members 40 of the diaper 1 may have the same length or may have different lengths.

Specifically, the crotch elastic region ER in the case where the string-like crotch elastic members 40 are arranged at intervals in the lateral direction is as follows. As shown in FIG. 5, the crotch elastic region ER refers to a region between a leftmost crotch elastic member 40a and a rightmost crotch elastic member 40b, the region being enclosed by a virtual line connecting longitudinal front-side ends of the stretchable portions of the crotch elastic members 40 arranged in the lateral direction and a virtual line connecting longitudinal back-side ends of the stretchable portions of the crotch elastic members 40. For example, as shown in FIG. 5, in the case where the five crotch elastic members 40 have the same length, the crotch elastic region ER has a rectangular shape. As shown in FIG. 6, in the case where, out of the five crotch elastic members 40, three crotch elastic members 40 located at the central portion are longer, a lateral central portion of the crotch elastic region ER protrudes outward in the longitudinal direction. In addition, in the case where the crotch elastic member 40 is a stretchy sheet (such as a stretchy nonwoven fabric sheet or a stretchy film), the region in which the stretchy sheet is arranged is defined as the crotch elastic region ER. In the case where a plurality of strip-shaped stretchy sheets or flat rubbers are arranged at intervals in the lateral direction, the same applies as in the case of string-like elastic members. That is, the region between a left end of the leftmost crotch elastic member 40 (strip-shaped stretchy sheet or flat rubber) and a right end of the rightmost crotch elastic member 40 is defined as the crotch elastic region ER.

However, as shown in FIG. 7, the crotch elastic members 40 that are arranged to be largely spaced apart from each other in the lateral direction are regarded as belonging to different crotch elastic regions ER. For example, the crotch elastic members 40 that are spaced apart from each other by at least three times the minimum spacing (Wa, in FIG. 7) between the plurality of crotch elastic members 40 arranged in the lateral direction are regarded as belonging to different crotch elastic regions ER. Accordingly, the crotch elastic region ER may be provided in the lateral central portion of the absorbent core 11 as shown in FIG. 5, or a plurality of (a pair of, in this case) crotch elastic regions ER may be provided at intervals in the lateral direction as shown in FIG. 7. In FIG. 5, the crotch elastic region ER is provided in a central portion CR obtained by dividing the absorbent core 11 into three equal regions at the maximum width of the absorbent core 11.

The crotch elastic members 40 overlapping the absorbent core 11 in the thickness direction are integrally joined to the absorbent core 11 with an adhesive or the like via the back sheet 13. Therefore, when the diaper 1 is worn, the crotch elastic members 40 contract in the longitudinal direction, and the absorbent core 11 is likely to deform so as to be pushed up toward the skin side and brought into close contact with the wearer's crotch portion. Accordingly, the close contact of the absorbent core 11 with the wearer's crotch portion is enhanced, thereby suppressing leakage of excrement. In addition, discomfort during wear can be reduced. Even after the absorbent core 11 absorbs excreted liquid and increases in weight, the absorbent core 11 is less likely to sag, and close contact with the wearer's crotch portion is maintained.

In particular, in the case where the crotch elastic region ER is provided in a lateral central portion CR of the absorbent core 11, the lateral central portion CR of the absorbent core 11 is deformed into an upwardly convex shape (toward the wearer's crotch portion). As a result, a gap is less likely to be formed between the wearer's excretion opening (for example, urethral opening) and the absorbent core 11, and it is possible to suppress leakage of excreted liquid such as urine that flows along the wearer's skin to the outside of the diaper 1.

### Specific-Color Region 60

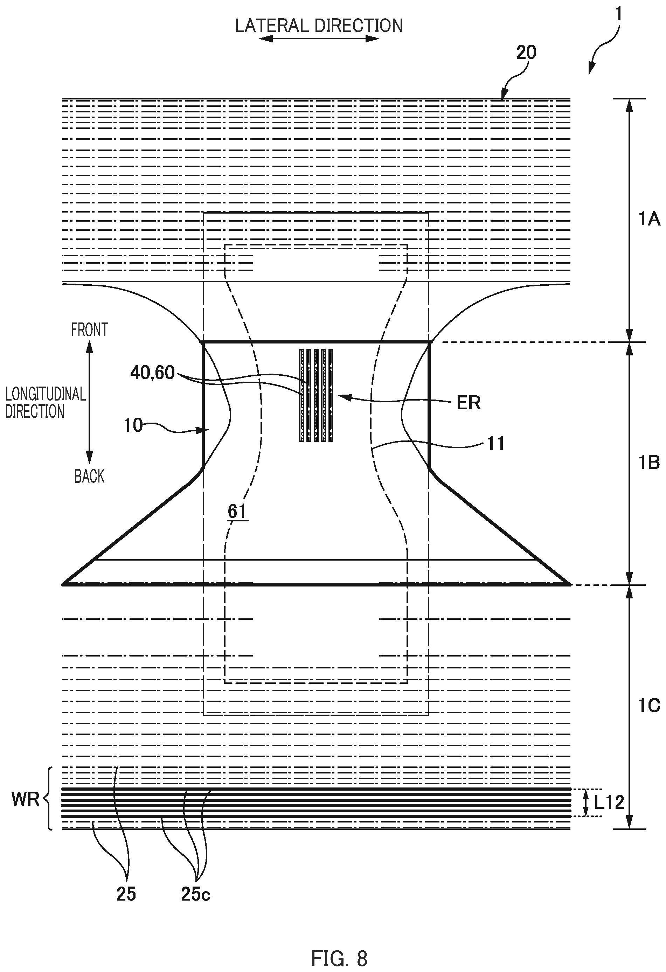

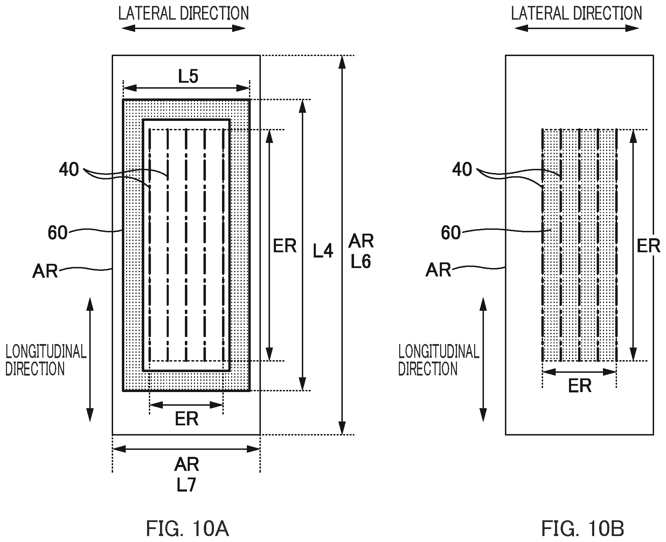

FIG. 8 is an illustrative view of a specific-color region 60. FIG. 9 is an enlarged view of the crotch elastic region ER and a peripheral region AR. FIGS. 10A, 10B, 11A, 11B, and 12 are illustrative views of a specific-color region 60 according to a modified example.

The diaper 1 includes the peripheral region AR that surrounds the crotch elastic region ER. In FIGS. 5 to 7, the crotch elastic region ER is stippled, and the peripheral region AR is hatched. The peripheral region AR is a region that, in the lateral direction, extends from a position ARa spaced apart leftward from a left end ERa of the crotch elastic region ER by a length Wa/2 equal to half a width Wa of the crotch elastic region ER, to a position ARb spaced apart rightward from a right end ERb of the crotch elastic region ER by the length Wa/2 equal to half the width Wa of the crotch elastic region ER, and that, in the longitudinal direction, extends from a position ARc spaced apart toward one side from a one-side (front side) end ERc of the crotch elastic region ER by a length equal to the width Wa of the crotch elastic region ER, to a position ARd spaced apart toward the other side from an other-side (back side) end ERd of the crotch elastic region ER by the length equal to the width Wa of the crotch elastic region ER. The peripheral region AR does not include the crotch elastic region ER.

However, when the width Wa of the crotch elastic region ER is large and the positions to be defined as the lateral ends ARa and ARb of the peripheral region AR are located outward beyond the lateral ends 10a and 10b of the absorbent main body 10, the lateral ends 10a and 10b of the absorbent main body 10 are defined as the lateral ends ARa and ARb of the peripheral region AR, respectively. Similarly, when the width Wa of the crotch elastic region ER is large and the positions to be defined as the longitudinal ends ARc and ARd of the peripheral region AR are located outward beyond the longitudinal ends 10c and 10d of the absorbent main body 10, the longitudinal ends 10c and 10d of the absorbent main body 10 are defined as the longitudinal ends ARc and ARd of the peripheral region AR, respectively. In other words, the peripheral region AR is defined so as to be contained within a range that overlaps the absorbent main body 10 in the thickness direction.

As described above, by providing the crotch elastic region ER in the crotch portion 1B of the diaper 1 (within the region outlined by a thick line in FIG. 8), close contact with the wearer's crotch portion is improved, making the diaper less likely to sag even after excretion. However, merely providing the crotch elastic members 40 in the diaper 1 may result in the user failing to notice the presence of the crotch elastic members 40. Then, as shown in FIGS. 1 and 8, the diaper 1 includes the "specific-color region 60" that can be visually recognized when the diaper 1 is viewed from the non-skin side.

When the diaper 1 is viewed from the non-skin side, the diaper 1 includes a background-color region 61 having a background color and the specific-color region 60 having a specific color different from the background color. The background-color region 61 is the region that occupies the largest area in the crotch portion 1B of the diaper 1 in the unfolded and stretched state. For example, in the case where the constituent materials of the absorbent main body 10 and the exterior member 20 located in the crotch portion 1B are white, the background color is white, and the white-colored region serves as the background-color region 61. Then, a region colored with a certain specific color (for example, blue or green) different from white serves as the specific-color region 60.

In the diaper 1 of the present embodiment, printing in a specific color is applied onto the back sheet 13, and a region colored with such a specific color is visually recognized from the non-skin side of the diaper 1 through the exterior member 20, thereby forming the specific-color region 60. In FIGS. 8 and 9, the specific-color regions 60, each being a long strip-like (rectangular) region extending in the longitudinal direction, are illustrated at positions overlapping the string-like crotch elastic members 40. Similar to the five crotch elastic members 40 arranged in the lateral direction, five rectangular specific-color regions 60 are arranged at intervals in the lateral direction.

Conversely, most of the materials located in the crotch portion 1B may be colored green, for example, and in this case, the region colored with green serves as the background-color region 61. Then, the region that is not colored with the background color (e.g., green), that is, the region in the base color of the material or the region colored with a color different from the background color, serves as the specific-color region 60.

There is no particular limitation on the method of coloring the material to form the specific-color region 60 and the background-color region 61. For example, the material may be colored by printing thereon or by incorporating a coloring agent into the material components (such as fibers). In the present embodiment, the back sheet 13 is colored by printing to form the specific-color region 60; however, the present invention is not limited thereto. Other materials such as the elastic-member cover sheet 17, the exterior member 20, and the crotch elastic member 40, may be colored to form the specific-color region 60 and/or the background-color region 61.

When the diaper 1 in the unfolded and stretched state is viewed from the non-skin side, the following applies. First, the ratio obtained by dividing the total area (A1) of the specific-color regions 60 provided in the crotch elastic region ER by the area (S1) of the crotch elastic region ER is defined as a "first area ratio (A1/S1)". The first area ratio is an area ratio of the specific-color regions 60 in the crotch elastic region ER. On the other hand, the ratio obtained by dividing the total area (A2) of the specific-color regions 60 provided in the peripheral region AR by the area (S2) of the peripheral region AR is defined as a "second area ratio A2/S2". The second area ratio is an area ratio of the specific-color regions 60 in the peripheral region AR.

At this time, in the diaper 1, the absolute value of the difference between the first area ratio and the second area ratio is equal to or greater than the first area ratio or is equal to or greater than the second area ratio. $\left|A1/S1-A2/S2\right| \geq A 1 / S 1 ,$ or $\left|A1/S1-A2/S2\right| \geq A 2 / S 2 .$

For example, in FIG. 9, within the crotch elastic region ER, there are three strip-shaped specific-color regions 60 and two strip-shaped specific-color regions 60 each having a half width. In contrast, only two strip-shaped specific-color regions 60 each having a half width are provided in the peripheral region AR. In other words, in FIG. 9, the specific-color regions 60 in the crotch elastic region ER have a larger area, and the first area ratio is large. On the other hand, the specific-color regions 60 in the peripheral region AR have a small area, and the second area ratio is small. Therefore, the absolute value of the difference between the first area ratio (large) and the second area ratio (small) is large and is equal to or greater than the second area ratio (small) (|A1/S1 - A2/S2| ≥ A2/S2). In other words, the "first area ratio" is approximately twice or more the "second area ratio". Thus, compared to the opposite case (where the absolute value of the difference is less than the second area ratio), the specific-color regions 60 provided in the crotch elastic region ER stand out. As a result, the user (such as the wearer or caregiver) who looks at the diaper 1 in its natural state before use is more likely to focus attention on the crotch elastic region ER, and is more likely to notice that the crotch portion 1B stretches and contracts (the presence of the crotch elastic member 40) due to wrinkles caused by contraction of the crotch elastic members 40.

Conversely, in FIG. 10A according to a modified example, a specific-color region 60 having a hollow rectangular shape (thick frame shape) is provided in the peripheral region AR so as to surround the crotch elastic region ER. In other words, the specific-color region 60 in the peripheral region AR is large, and the second area ratio is large. On the other hand, the specific-color region 60 is not provided in the crotch elastic region ER, and the first area ratio is small (zero, in this case). Therefore, the absolute value of the difference between the first area ratio (small) and the second area ratio (large) is large and is equal to or greater than the first area ratio (small) (|A1/S1 - A2/S2| ≥ A1/S1). In other words, the second area ratio is approximately twice or more the first area ratio. Thus, compared to the opposite case (where the absolute value of the difference is less than the first area ratio), the specific-color region 60 in the peripheral region AR stands out, and the crotch elastic region ER surrounded by the specific-color region 60 stands out. As a result, when the user looks at the diaper 1 in its natural state before use, the user is more likely to focus attention on the crotch elastic region ER, and is more likely to notice that the crotch portion 1B stretches and contracts (the presence of the crotch elastic members 40) due to wrinkles caused by contraction of the crotch elastic members 40.

Before using the diaper 1, the user who notices the presence of the crotch elastic members 40 can recognize that the diaper 1 is a product having good close contact with the wearer's crotch portion and in which the absorbent core 11 is unlikely to sag. Thus, it is possible to alleviate the user's concern that the absorbent core 11 may sag when pressure is applied by the inner thighs or when the weight of the absorbent core 11 increases due to excreted fluid. Accordingly, the user's concern about leakage caused by sagging of the diaper 1 and an impression that the diaper 1 is uncomfortable to wear can be eliminated. This allows the user to use the diaper 1 with peace of mind. Furthermore, the wearer's recognition of the presence of the crotch elastic members 40 makes the wearer more likely to perceive good close contact with the crotch portion during wear of the diaper 1, thereby reducing the concern about leakage.

In FIGS. 9 to 12, the specific-color region 60 is exemplified. However, the shape, number, arrangement, and the like of the specific-color region 60 are not particularly limited, as long as the relationship between the area ratios is satisfied.

More preferably, the absolute value of the difference between the first area ratio and the second area ratio is equal to or greater than twice the first area ratio or is equal to or greater than twice the second area ratio. $\left|A1/S1-A2/S2\right| \geq 2 \times A 1 / S 1 ,$ or $\left|A1/S1-A2/S2\right| \geq 2 \times A 2 / S 2 .$

Accordingly, the difference between the area ratio of the specific-color regions 60 in the crotch elastic region ER and the area ratio of the specific-color regions 60 in the peripheral region AR becomes greater. Therefore, the crotch elastic region ER provided with the specific-color region 60 is more likely to stand out, or the crotch elastic region ER surrounded by the specific-color region 60 is likely to stand out more prominently. As a result, the user is more likely to notice the presence of the crotch elastic members 40.

The first area ratio and the second area ratio can be measured by a known method. For example, the diaper 1 in an unfolded and stretched state is fixed with the non-skin-side surface facing upward, and ranges of the crotch elastic region ER, the peripheral region AR, and the specific-color regions 60 in the diaper 1 are identified. The area ratio can be determined by actually measuring the lengths of the target regions (ER, AR, 60) and calculating the areas thereof. Alternatively, the area ratio can be obtained by acquiring image data of the diaper 1 and obtaining the areas of the target regions (ER, AR, 60) using an area calculation program.

Furthermore, the background color and the specific color being different means that the colors only need to be different to such an extent that a plurality of subjects (for example, ten people) can visually recognize the difference in color. Specifically, when one of the specific color and the background color is white and the other is a chromatic color other than white, the colors are considered to be different. Preferably, one of the specific color and the background color is included in white as defined below, and the other is a chromatic color not included in white as defined below. The term "white" includes, for example, a color represented by "N9.5" in the Munsell color system, a color represented by "255, 255, 255" in the RGB color space (so-called pure white), and also includes colors having a color difference of less than 12 from pure white. The color difference for the white color can be measured using, for example, a color difference measuring instrument such as CR-300 manufactured by Konica Minolta Inc. or a meter equivalent thereto. As a light source of the measuring instrument, a C light source (a standard of the International Commission on Illumination (CIE)) can be used. The diameter of the measurement window of the measuring instrument in this case is 40 mm. The color difference is represented by ΔL, Δa, and Δb in the (L*a*b*) color space with respect to the white reference plate. The color difference with respect to the white reference plate can be calculated by measuring the Lab value of the white reference plate and the Lab value of the target region, respectively (the diaper 1 in the unfolded and stretched state with the non-skin-side surface thereof facing upward is measured). The white reference plate is set as Lab0 at (X93.19, Y95.20, Z112.28). Here, L is a value indicating brightness, and a and b are the plane coordinates of chromaticity. When the Lab values of the white reference plate are represented by L0, a0, and b0, and when the Lab values of the target region are represented by L1, a1, and b1, the color difference can be calculated by ΔL = L0 - L1, Δa = a0 - a1, and Δb = b0 - b1.

In addition, both the specific color and the background color may be chromatic colors other than white. In this case, even when the colors belong to the same color system (e.g., the blue color system), in the case where the colors differ in brightness (e.g., blue and light blue) and a plurality of subjects can visually recognize the difference in color, the colors are considered different. Preferably, the determination is made using a Munsell hue circle divided into 20 hues. First, a plurality of subjects (for example, ten people) look at the diaper 1 in the unfolded and stretched state from the non-skin side, and determine which hue on the Munsell hue circle each of the target regions (the specific-color region 60 and the background-color region 61) corresponds to, that is, which hue is closest to each of the target regions. The hue chosen by the greatest number of people is defined as the hue of the target region. When the hue of the specific-color region 60 is separated from the hue of the background-color region 61 by two or more hues (when the hue of the specific-color region 60 is neither the same as nor adjacent to the hue of the background-color region 61), the colors are considered to be different.

In addition, in the unfolded and stretched state of the diaper 1, it is preferable that at least one of the crotch elastic region ER and the peripheral region AR includes the specific-color region 60 in which a longitudinal length is longer than a lateral length. For example, in FIG. 9, in the crotch elastic region ER, a plurality of rectangular specific-color regions 60 in which a longitudinal length L1 is longer than a lateral length L2 are arranged in the lateral direction. In FIG. 10A according to the modified example, the peripheral region AR includes a specific-color region 60 having a hollow rectangular shape (thick frame shape), the specific-color region 60 in which a longitudinal length L4 is longer than a lateral length L5.

As described above, since the specific-color region 60 (a figure formed by the specific color) is long in the longitudinal direction, the user who looks at this configuration is more likely to get the impression that the crotch elastic members 40 stretch and contract in the longitudinal direction. Therefore, the user is more likely to recognize that the diaper 1 is a product in which the crotch elastic members 40 maintain a state in which the absorbent core 11 is pushed up toward the skin side, and that the absorbent article has good close contact with the wearer's crotch portion and is less likely to sag.

Furthermore, as shown in FIG. 9, in the unfolded and stretched state of the diaper 1, it is preferable that, in the crotch elastic region ER, a plurality of specific-color regions 60 that each have a strip shape and extend along the longitudinal direction are arranged in the lateral direction. Accordingly, the width L2 of each strip-shaped specific-color region 60 is reduced, and the specific-color region 60 becomes more elongated. The user who looks at this configuration is more likely to get the impression that the crotch elastic members 40 stretch and contract in the longitudinal direction. Therefore, the user is more likely to recognize that the diaper 1 is a product that has good close contact with the wearer's crotch portion and that is less likely to sag.

However, the present invention is not limited to the above. For example, as shown in FIG. 10B according to a modified example, the entire crotch elastic region ER may be the specific-color region 60. Only a single rectangular specific-color region 60 that is elongated in the longitudinal direction may be provided in the crotch elastic region ER.

In addition, in the unfolded and stretched state of the diaper 1, it is preferable that, in at least one of the longitudinal direction and the lateral direction, the total length of the specific-color region 60 provided in one of the crotch elastic region ER and the peripheral region AR is equal to or greater than half the length of the one of the crotch elastic region ER and the peripheral region AR.

For example, in FIG. 9, the longitudinal length L1 of the specific-color region 60 provided in the crotch elastic region ER matches the longitudinal length L1 of the crotch elastic region ER and is equal to or greater than half the longitudinal length of the crotch elastic region ER. In this case, the specific-color regions 60 are provided over a wide range of the crotch elastic region ER in the longitudinal direction. As a result, the user is more likely to focus attention on the crotch elastic region ER. Furthermore, in FIG. 9, the total lateral length of the specific-color regions 60 provided in the crotch elastic region ER (specifically, the sum of the lengths of three strip-shaped specific-color regions 60 (L2 × 3) and the lengths of two specific-color regions 60 each having a half width (L2/2 × 2)) is equal to or greater than half the lateral length L3 of the crotch elastic region ER. Thus, the specific-color regions 60 are provided over a wide range of the crotch elastic region ER in the lateral direction. As a result, the user is more likely to focus attention on the crotch elastic region ER.

As described above, since the length relationship is satisfied in both the longitudinal direction and the lateral direction, the user is more likely to focus attention on the crotch elastic region ER. As a result, the user is more likely to notice the presence of the crotch elastic members 40. In addition, the user can recognize a range corresponding to at least half the position where the crotch elastic members 40 actually stretch and contract. However, the present invention is not limited to the above, and the above-described length relationship only needs to be satisfied in only one of the longitudinal direction and the lateral direction.

In FIG. 10A, a longitudinal length L4 of the specific-color region 60 provided in the peripheral region AR is equal to or greater than half a longitudinal length L6 of the peripheral region AR (L4 ≥ L6/2). Further, a lateral length L5 of the specific-color region 60 is equal to or greater than half the lateral length L7 of the peripheral region AR (L5 ≥ L7/2). As a result, the specific-color region 60 is provided over a wide range of the peripheral region AR in the longitudinal direction and the lateral direction, making the user likely to focus attention on the crotch elastic region ER.

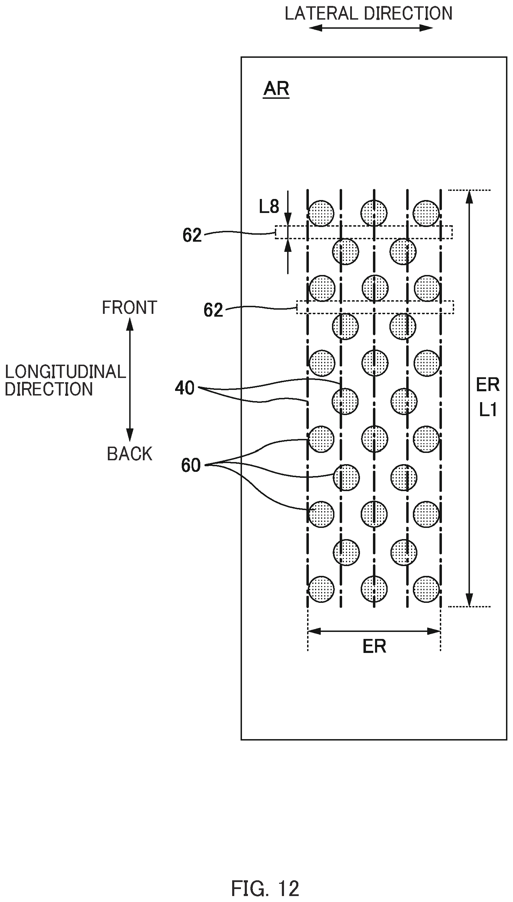

The specific-color region 60 is not limited to a region that is long and continuous in the longitudinal direction and the lateral direction. A plurality of relatively small specific-color regions 60 may be arranged in the longitudinal direction and the lateral direction. For example, in FIG. 12, in the crotch elastic region ER, circular specific-color regions 60 are arranged in a staggered pattern at intervals in the longitudinal direction and the lateral direction. Even in this case, as long as the above-described relationship between the area ratios is satisfied, the crotch elastic region ER stands out, thereby making the user more likely to notice the presence of the crotch elastic members 40.

In the case as shown in FIG. 12, a region where the specific-color regions 60 are not provided over the entire crotch elastic region ER in the lateral direction or the longitudinal direction is defined as a non-specific-color region 62. Then, the total length of the regions excluding the non-specific-color region 62 in one of the crotch elastic region ER and the peripheral region AR is defined as the total length of the specific-color regions 60 provided in the one of the crotch elastic region ER and the peripheral region AR. For example, in the longitudinal direction shown in FIG. 12, the length obtained by subtracting the total length of the number "n" of the non-specific color regions 62 (L8 × n) from the longitudinal length L1 of the crotch elastic region ER is the total length of the specific-color regions 60 provided in the crotch elastic region ER, that is, the total length of the plurality of specific-color regions 60 arranged in a staggered pattern in the longitudinal direction. It is preferable that the total length is equal to or greater than half the length L1 of the crotch elastic region ER.

It is preferable that the first area ratio is equal to or greater than 1/2, or the second area ratio is equal to or greater than 1/2. By having the first area ratio be equal to or greater than 1/2, at least half of the crotch elastic region ER becomes the specific-color region 60. Therefore, compared to the case where the first area ratio is less than 1/2, the crotch elastic region ER stands out, making the user likely to focus attention on the crotch elastic region ER and to notice the presence of the crotch elastic members 40. Similarly, by having the second area ratio be equal to or greater than 1/2, at least half of the peripheral region AR becomes the specific-color region 60. Therefore, compared to the case where the second area ratio is less than 1/2, a larger portion of the crotch elastic region ER is surrounded by the specific-color region 60, making the user likely to focus attention on the crotch elastic region ER and to notice the presence of the crotch elastic members 40.

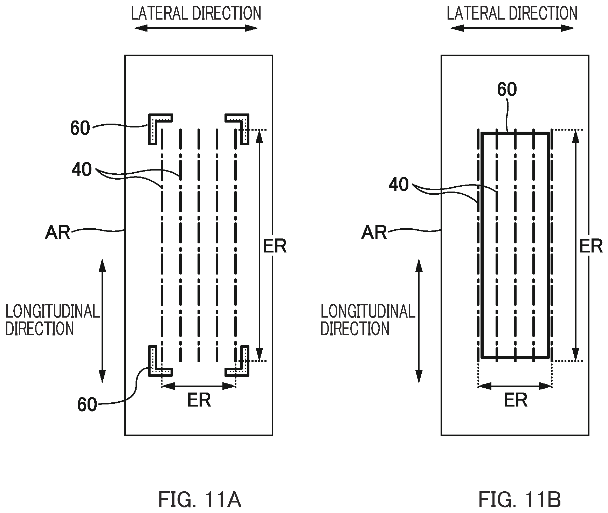

However, the present invention is not limited to the above. The first area ratio or the second area ratio may be less than 1/2. For example, in FIG. 11A, the area of the specific-color region 60 provided in the peripheral region AR is small, and the second area ratio is less than 1/2. In this case as well, when the first area ratio is small (in FIG. 11A, since the first area ratio is zero), the aforementioned relationship of the area ratios (|A1/S1 - A2/S2| ≥ A1/S1) is satisfied, and the specific-color region 60 makes the user more likely to focus attention on the crotch elastic region ER. Further, in FIG. 11A, specific-color regions 60 each having a corner-bracket shape are provided outside the four corners of the crotch elastic region ER, respectively, and indicate the positions of the corners of the crotch elastic region ER. Therefore, even when the second area ratio is less than 1/2, the position of the crotch elastic region ER is indicated, making the user likely to focus attention on the crotch elastic region ER.

As shown in FIG. 11B, a specific-color region 60 provided in the crotch elastic region ER may have a thin line-like frame shape, and the first area ratio may be less than 1/2. In this case as well, when the second area ratio is small (in FIG. 11B, since the second area ratio is zero), the aforementioned relationship of the area ratios (|A1/S1 - A2/S2| ≥ A2/S2) is satisfied. The specific-color region 60 in FIG. 11B has a frame shape that is substantially similar to the outline of the crotch elastic region ER. Therefore, even when the first area ratio is less than 1/2, the position of the crotch elastic region ER is indicated, making the user likely to focus attention on the crotch elastic region ER.

In a modified example shown in FIG. 12, specific-color regions 60 are provided in the crotch elastic region ER, but not provided in the peripheral region AR. In this case, the user is more likely to focus attention directly on the crotch elastic region ER, and, due to wrinkles caused by contraction of the crotch elastic members 40, the user is more likely to notice the presence of the crotch elastic members 40.

In the crotch elastic region ER, in the case where a plurality of string-like crotch elastic members 40 are arranged in the lateral direction, as shown in FIGS. 9, 10B, and 12, it is preferable that, in the unfolded and stretched state of the diaper 1, a stretchable portion 40E of the crotch elastic member 40 has a portion that overlaps the specific-color region 60 in the thickness direction. Accordingly, the user is more likely to focus attention directly on the stretchable portion 40E of the crotch elastic member 40, making the user likely to notice the presence of the crotch elastic members 40 due to wrinkles caused by contraction of the crotch elastic members 40. In particular, in the case where the specific color is a chromatic color other than white, wrinkles stand out more. Furthermore, in the case where the color of the specific-color region 60 differs from that of the crotch elastic members 40, the visibility of the crotch elastic members 40 is enhanced. Therefore, the user is more likely to notice the presence of the crotch elastic members 40.

Only a part of the stretchable portion 40E of the crotch elastic member 40 only needs to overlap the specific-color region 60, but preferably, most of the stretchable portion 40E of the crotch elastic member 40 overlaps the specific-color region 60 in the thickness direction. Accordingly, the user can identify the position where the crotch elastic members 40 actually stretch and contract.

In FIG. 9, five strip-shaped specific-color regions 60 are provided in the lateral direction, similarly to the five crotch elastic members 40 arranged in the lateral direction. Therefore, the user who looks at the specific-color region 60 can intuitively recognize that the plurality of string-like crotch elastic members 40 are arranged in the lateral direction.

On the other hand, in the unfolded and stretched state of the diaper 1, in the region outside the crotch elastic region ER in the longitudinal direction, a non-stretchable portion 40N of the crotch elastic member 40 preferably has a portion that does not overlap the specific-color region 60 in the thickness direction. Only a part of the non-stretchable portion 40N of the crotch elastic member 40 is sufficient to not overlap the specific-color region 60, but preferably, as shown in FIG. 9, the entire non-stretchable portion 40N of the crotch elastic member 40 does not overlap the specific-color region 60.

According to the above, it is possible to prevent the user from mistakenly recognizing that the non-stretchable portion 40N of the crotch elastic member 40 overlapping the specific-color region 60 stretches and contracts, thereby enabling the user to correctly identify the position where actual stretching and contraction occur. In the manufacturing process of the diaper 1, by cutting the portion of the crotch elastic member 40 that is not fixed to the material, the non-stretchable portion 40N of the crotch elastic member 40 is formed. Therefore, the non-stretchable portion 40N of the crotch elastic member 40 is not fixed to the material, and thus can freely contract and is likely to bend. Since the non-stretchable portion 40N of the crotch elastic member 40 does not overlap the specific-color region 60, the non-stretchable portion 40N of the crotch elastic member 40 that is not aligned along the longitudinal direction is less likely to stand out. As a result, it is possible to suppress reduction in the impression that the crotch elastic members 40 stretch and contract in the longitudinal direction.

In addition, deterioration in the appearance (aesthetic quality) of the diaper 1 can also be suppressed.

FIG. 13A is a partial cross-sectional view of the absorbent main body 10, and FIG. 13B is an illustrative view of joining portions 70 between the back sheet 13 and the elastic-member cover sheet 17. As shown in FIG. 4 described above, the absorbent main body 10 includes the liquid-impermeable back sheet 13 provided on the non-skin side with respect to the absorbent core 11, and the elastic-member cover sheet 17 (non-skin-side sheet) provided on the non-skin side with respect to the back sheet 13. The crotch elastic members 40 are provided between the back sheet 13 and the elastic-member cover sheet 17. In the diaper 1 of the present embodiment, the specific-color region 60 is formed by coloring the back sheet 13. In this case, as shown in FIGS. 13A and 13B, it is preferable that, in the unfolded and stretched state of the diaper 1, the joining portions 70 that join the back sheet 13 and the elastic-member cover sheet 17 are provided between the plurality of string-like crotch elastic members 40 arranged in the lateral direction. Examples of the joining method for the joining portions 70 are not particularly limited and may include a joining method using an adhesive.

In the portions where the string-like crotch elastic members 40 are arranged, the back sheet 13 and the elastic-member cover sheet 17 are joined via the crotch elastic members 40. However, in the case where the joining portions 70 are not provided between the crotch elastic members 40, the elastic-member cover sheet 17 is likely to lift away from the back sheet 13. Thus, by providing the joining portions 70, lifting of the elastic-member cover sheet 17 relative to the back sheet 13 can be suppressed, thereby enhancing the visibility of the specific-color region 60 formed in the back sheet 13. As a result, the crotch elastic region ER stands out, making the user likely to recognize the presence of the crotch elastic members 40.

The joining portions 70 illustrated in FIG. 13B each extend continuously in the longitudinal direction over the entire crotch elastic region ER, but the pattern of the joining portions 70 is not particularly limited. For example, the joining portions 70 may be intermittently arranged in the longitudinal direction, or the joining portions 70 may be provided only in a part of the crotch elastic region ER in the longitudinal direction. In addition, in the case where three or more of the crotch elastic members 40 are arranged, a joining portion 70 is simply required to be provided in at least one pair of adjacent crotch elastic members 40 arranged in the lateral direction.

As shown in FIG. 13B, it is preferable that the joining portions 70 that join the back sheet 13 and the elastic-member cover sheet 17 at positions between the plurality of crotch elastic members 40 arranged in the lateral direction, extend outward beyond the crotch elastic region ER in the longitudinal direction.

As described above, the non-stretchable portions 40N of the crotch elastic members 40 are not fixed to the material, and can freely contract and are likely to bend. Thus, by providing the joining portions 70 as described above, bending of the non-stretchable portions 40N of the crotch elastic members 40 in the lateral direction is restricted by the joining portions 70. In particular, as shown in FIG. 13B, by providing the joining portions 70 on both lateral sides of the non-stretchable portion 40N of each crotch elastic member 40, the non-stretchable portion 40N of the crotch elastic member 40 is accommodated between the joining portions 70 arranged in the lateral direction, thereby suppressing large bending. As a result, it is possible to suppress reduction in the impression of the crotch elastic members 40 that stretch and contract in the longitudinal direction. In addition, deterioration in the appearance (aesthetic quality) of the diaper 1 can also be suppressed.

In the present embodiment, the specific-color region 60 is formed by coloring the back sheet 13 by printing, but the present invention is not limited thereto. The crotch elastic members 40 may be colored to form the specific-color region 60. That is, by coloring the crotch elastic members 40 in a color different from the background color, the region in which the crotch elastic members 40 are arranged becomes the specific-color region 60 when the diaper 1 is viewed from the non-skin side.

In this case, the user is more likely to focus attention directly on the crotch elastic members 40, and, due to wrinkles caused by contraction of the crotch elastic members 40, the user is more likely to notice the presence of the crotch elastic members 40. In addition, the user can correctly identify the position where actual stretching and contraction occur. In particular, in the case where the crotch elastic member 40 is a sheet-shaped elastic member or the case where a plurality of strip-shaped elastic members are arranged in the lateral direction, the first area ratio increases (the area of the specific-color region 60 in the crotch elastic region ER increases), and the aforementioned relationship of the area ratios is more likely to be satisfied.

As shown in FIG. 2, the diaper 1 includes a waist elastic region WR that stretches and contracts along the lateral direction at least at a longitudinal one-end portion of the diaper 1, here, at each of two front-side and back-side end portions. In the waist elastic region WR, the longitudinal spacing of the waist elastic members 24 and 25 is smaller than that in a region on the crotch-side with respect to the waist elastic region WR. It is preferable that, when the diaper 1 in the unfolded and stretched state is viewed from the non-skin side, the waist elastic region WR includes a waist colored region 25c colored along the lateral direction. That is, the waist colored region 25c is a region that is colored in a color different from that of a peripheral region (such as the background-color region 61) and is visually recognized as having a color different from that of the peripheral region when the diaper 1 is viewed from the non-skin side. The definition of different colors is the same as that of the specific-color region 60 and the background-color region 61. In the diaper 1 of the present embodiment, as shown in FIG. 8, the waist colored region 25c is formed by coloring some of the waist elastic members 25 belonging to the back-side waist elastic region WR (the third to eighth waist elastic members 25 counted from the waist side).

According to the above, since the waist colored regions 25c stand out, the user is more likely to focus attention on the waist elastic region WR and is more likely to notice that the waist portion stretches and contracts (the presence of the waist elastic members 25). Thus, before using the diaper 1, the user can recognize that the diaper 1 is a product that has good close contact with the wearer's waist portion, thereby alleviating the concern about leakage through a gap and eliminating the impression that the diaper 1 is uncomfortable to wear. This allows the user to use the diaper 1 with peace of mind.

In the diaper 1, the waist colored region 25c is provided only in the back-side waist elastic region WR. Therefore, it is also possible to determine the front side and back side of the diaper 1 based on the waist colored regions 25c. However, the present invention is not limited to the above, but the waist colored region may be provided only in the front-side waist elastic region WR, or the waist colored region may be provided in both the back-side and front-side waist elastic regions WR.

As shown in FIGS. 2 and 4, the diaper 1 includes side-portion elastic regions SR provided in two lateral side portions of the absorbent main body 10, the side-portion elastic regions SR stretching and contracting along the longitudinal direction. The diaper 1 of the present embodiment includes: side-portion elastic regions SR each delimited by the stretchable portions of the leak-proof-wall elastic members 51 that are located on the skin side with respect to the top sheet 12 and cause the leak-proof-wall portions 50 to rise toward the skin side; and side-portion elastic regions SR each delimited by the stretchable portions of the leg elastic members 52 that are located outward in the lateral direction with respect to the leak-proof-wall elastic members 51. It is preferable that, when the diaper 1 in the unfolded and stretched state is viewed from the non-skin side or the skin side, the side-portion elastic region SR includes a side-portion colored region 51c that is colored along the longitudinal direction. That is, the side-portion colored region 51c is a region that is colored in a color different from that of a peripheral region (such as the background-color region 61 and the skin-side surface of the absorbent main body 10) and is visually recognized as having a color different from that of the peripheral region when the diaper 1 is viewed from the non-skin side or the skin side. The definition of different colors is the same as that of the specific-color region 60 and the background-color region 61. In the diaper 1 of the present embodiment, as shown in FIG. 4, the side-portion colored region 51c is formed by coloring some of the leak-proof-wall elastic members 51 (the first and second leak-proof-wall elastic members 51 counted from the inner side in the lateral direction).

According to the above, since the side-portion colored regions 51c stand out, the user is more likely to focus attention on the side-portion elastic regions SR and is more likely to notice that leg portions stretch and contract (the presence of the leak-proof-wall elastic members 51 and the like). As a result, before using the diaper 1, the user can recognize that the diaper 1 is a product that has good fit around the leg portions of the wearer, thereby alleviating the concern about leakage through gaps and eliminating the impression that the diaper 1 is uncomfortable to wear. This allows the user to use the diaper 1 with peace of mind.

However, the present invention is not limited to the above. A side-portion colored region may be formed by the leg elastic members 52 being colored, or may be formed by both the leak-proof-wall elastic members 51 and the leg elastic members 52 being colored.

In addition, the waist elastic region WR and the side-portion elastic region SR may be formed not only by string-like elastic members but also by sheet-shaped or strip-shaped elastic members. In addition, the waist colored region and the side-portion colored region are not limited to being formed by coloring elastic members such as the waist elastic members 25 and the leak-proof-wall elastic members 51, and may be formed by coloring a material (such as the exterior member 20 and the side sheet 16) that overlaps the elastic members in the thickness direction.

In addition, it is preferable that the specific-color region 60 in the crotch elastic region ER as viewed from the non-skin side of the diaper 1, the waist colored region 25c as viewed from the non-skin side of the diaper 1, and the side-portion colored region 51c as viewed from the non-skin side or the skin side of the diaper 1 are similar in color. The side-portion colored region 51c provided in the side-portion elastic region SR of each leak-proof-wall portion 50 has higher visibility as viewed from the skin side than viewed from the non-skin side of the diaper 1.

By providing the colored regions that are similar in color in the three elastic regions (the crotch elastic region ER, the waist elastic region WR, and the side-portion elastic region SR) of the diaper 1, a sense of visual unity is created among the three elastic regions. Therefore, the user is more likely to recognize the three elastic regions, and can also visually and intuitively recognize that the crotch portion, the waist portion, and the leg portions of the diaper 1 each stretch and contract. Furthermore, due to the three elastic regions that are similar in color, the user is more likely to recognize that the diaper 1 fits all around the wearer (particularly that the fit is good at regions where leakage is likely to occur), thereby allowing the user to use the diaper 1 with peace of mind. The appearance (aesthetic quality) of the diaper 1 is also improved.

In the case where the three elastic regions (ER, WR, and SR) fall within the aforementioned definition of white, it can be said that the three elastic regions have similar white colors. Furthermore, in the case where the colors of the three elastic regions are chromatic colors other than white, similar colors can be determined using the Munsell hue circle divided into 20 hues. First, a plurality of subjects (for example, ten people) look at the diaper 1 in the unfolded and stretched state from either the non-skin side or the skin side, and determine which hue on the Munsell hue circle corresponds to each of the target regions (ER, WR, SR), that is, which hue is closest to each of the target regions. The hue chosen by the greatest number of people is defined as the hue of the target region. When the respective hues of the three elastic regions (ER, WR, SR) are the same as one another or adjacent to one another, the colors of the three elastic regions are considered to be similar.

In the diaper 1 in the unfolded and stretched state, it is preferable that the maximum lateral width L2 of the specific-color region 60 in the crotch elastic region ER (see FIG. 9) is larger than the maximum longitudinal width of the waist colored region 25c (the diameter of the colored waist elastic member 25, see FIG. 8) and the maximum lateral width of the side-portion colored region 51c (the diameter of the colored leak-proof-wall elastic member 51, see FIG. 4).

As a result, among the three elastic regions, the crotch elastic region ER stands out the most, making the user likely to focus attention on the crotch elastic region ER and to notice the presence of the crotch elastic members 40. Therefore, before using the diaper 1, the user can recognize that the diaper 1 is a product that has good close contact with the crotch portion and is unlikely to sag. This allows the user to use the diaper 1 with peace of mind.

Preferably, in the unfolded and stretched state of the diaper 1, a length L11 from the left end to the right end of the specific-color region 60 provided in the crotch elastic region ER and the peripheral region AR (see FIG. 9) is longer than a length L12 from a longitudinal one-side end to a longitudinal other-side end of the waist colored region 25c provided in the waist elastic region WR (see FIG. 8), and is also longer than a lateral length L13 from the left end to the right end of the side-portion colored region 51c provided in the side-portion elastic region SR. In this case as well, among the three elastic regions, the crotch elastic region ER stands out the most, making the user likely to notice the presence of the crotch elastic members 40.

### Second Embodiment

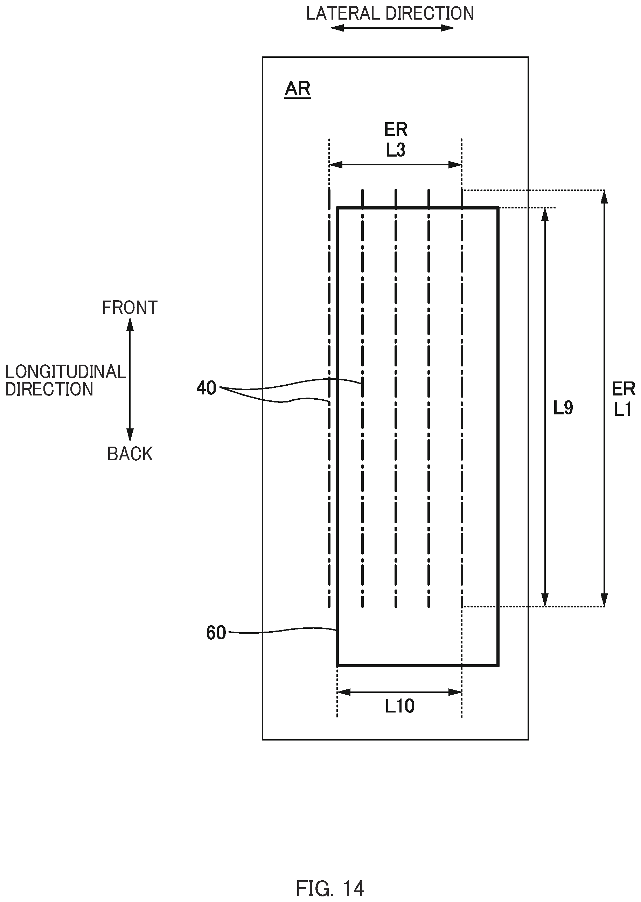

FIG. 14 is an illustrative view of the specific-color region 60 in the diaper 1 of the second embodiment. In the second embodiment, points different from the first embodiment will be mainly described. In the diaper 1 of the second embodiment, similarly to the diaper 1 of the first embodiment, at least a part of the crotch elastic region ER is provided in the crotch portion 1B that is the central region obtained by dividing the diaper 1 into three equal regions in the longitudinal direction. As illustrated in FIGS. 5 to 7, the diaper 1 includes the peripheral region AR that surrounds the crotch elastic region ER. When the diaper 1 of the second embodiment is viewed from the non-skin side, the diaper 1 includes the background-color region 61 and the specific-color region 60 having a specific color that is different from the background color. The background-color region 61 is a region that occupies the largest area in the crotch portion 1B of the diaper 1.

In the diaper 1 of the second embodiment, in the unfolded and stretched state of the diaper 1, in at least one of the longitudinal direction and the lateral direction, the total length of the specific-color regions 60 provided in one of the crotch elastic region ER and the peripheral region AR is equal to or greater than half the length of the one of the regions.

For example, in FIG. 14, a narrow frame-shaped specific-color region 60 following the outline of the crotch elastic region ER is positioned slightly offset from the crotch elastic region ER. In this case, the specific-color region 60 is provided in both the crotch elastic region ER and the peripheral region AR with substantially the same area. Therefore, the relationship of the area ratios in the first embodiment (|A1/S1 - A2/S2| ≥ A1/S1, or |A1/S1 - A2/S2| ≥ A2/S2) may fail to be satisfied. However, a longitudinal length L9 of the specific-color region 60 provided in the crotch elastic region ER is equal to or greater than half the longitudinal length L1 of the crotch elastic region ER (L9 ≥ L1/2). Further, a lateral length L10 of the specific-color region 60 provided in the crotch elastic region ER is equal to or greater than half the lateral length L3 of the crotch elastic region ER (L10 ≥ L3/2).

Thus, by providing the specific-color region 60 over a wide range of the crotch elastic region ER in the longitudinal direction and the lateral direction, the user is more likely to focus attention on the crotch elastic region ER. As a result, due to wrinkles caused by contraction of the crotch elastic members 40, the user is more likely to notice the presence of the crotch elastic members 40. Therefore, even before using the diaper 1, the user can recognize that the diaper 1 is a product that has good close contact with the wearer's crotch portion and is unlikely to sag. This allows the user to use the diaper 1 with peace of mind. Although not shown, the one of the regions may be the peripheral region AR. Even when the specific-color region 60 is provided over a wide range of the peripheral region AR in at least one of the longitudinal direction and the lateral direction, the same effects can be obtained.

In addition, the specific-color region 60 may be formed by coloring the string-like crotch elastic members 40. In this case, since the string-like crotch elastic members 40 are thin, the area of the specific-color region 60 provided in the crotch elastic region ER becomes small. In the case where the non-stretchable portions 40N of the crotch elastic members 40 located in the peripheral region AR are also colored, the difference between the first area ratio and the second area ratio becomes smaller. Therefore, the relationship of the area ratios of the first embodiment may fail to be satisfied. However, the longitudinal length of the colored string-like crotch elastic member 40, that is, the longitudinal length of the specific-color region 60, is equal to or greater than half the longitudinal length of the crotch elastic region ER. As a result, the user is more likely to focus attention on the crotch elastic region ER and to notice the presence of the crotch elastic members 40.

### Others

The above-described embodiment is intended to facilitate the understanding of the present invention and is not intended to limit the interpretation of the present invention. The present invention can be modified or improved without departing from the spirit thereof, and additionally, it is needless to say that equivalents thereof are included in the present invention.

### [REFERENCE SIGNS LIST]

1: underpants-shaped disposable diaper (absorbent article)
2: waist joining portion
10: absorbent main body
11: absorbent core, 12: top sheet
13: back sheet
16: side sheet
17: elastic-member cover sheet (non-skin-side sheet)
20: exterior member
21: front-side outer-layer sheet, 22: back-side outer-layer sheet
23: inner-layer sheet
24: (front-side) waist elastic member
25: (back-side) waist elastic member
26: buttocks elastic member
31: cover sheet, 32: cover sheet
40: crotch elastic member
50: leak-proof-wall portion
51: leak-proof-wall elastic member (side-portion elastic member)
52: leg elastic member
60: specific-color region 61: background-color region
70: joining portion
ER: crotch elastic region, AR: peripheral region

## Claims

1. An absorbent article having, in an unfolded and stretched state, a longitudinal direction, a lateral direction, and a thickness direction that intersect one another,
the absorbent article comprising:
an absorbent core;
a crotch elastic region delimited by a stretchable portion of a crotch elastic member; and
a peripheral region surrounding the crotch elastic region,
the crotch elastic member being provided on a non-skin side with respect to the absorbent core,
the crotch elastic member stretching and contracting along the longitudinal direction,
at least a part of the crotch elastic region being provided in a crotch portion corresponding to a central region,
the central region being obtained by dividing the absorbent article into three equal regions in the longitudinal direction,
the peripheral region being a region that, in the lateral direction, extends from a position spaced apart leftward from a left end of the crotch elastic region by a length equal to half a width of the crotch elastic region, to a position spaced apart rightward from a right end of the crotch elastic region by the length equal to half the width of the crotch elastic region, and that, in the longitudinal direction, extends from a position spaced apart toward one side from a one-side end of the crotch elastic region by a length equal to the width of the crotch elastic region, to a position spaced apart toward another side from an other-side end of the crotch elastic region by the length equal to the width of the crotch elastic region,
when the absorbent article is viewed from the non-skin side,
the absorbent article including:
a background-color region having a background color; and
a specific-color region having a specific color different from the background color,
the background-color region being a region that occupies the largest area in the crotch portion,
a first area ratio being a ratio obtained by dividing a total area of the specific-color region provided in the crotch elastic region by an area of the crotch elastic region,
a second area ratio being a ratio obtained by dividing a total area of the specific-color region provided in the peripheral region by an area of the peripheral region,
an absolute value of a difference between the first area ratio and the second area ratio being equal to or greater than the first area ratio, or being equal to or greater than the second area ratio.

2. The absorbent article according to claim 1, wherein
in an unfolded and stretched state, the specific-color region in which a longitudinal length is longer than a lateral length is provided in at least one of the crotch elastic region and the peripheral region.

3. The absorbent article according to claim 2, wherein
in an unfolded and stretched state, in the crotch elastic region, a plurality of the specific-color regions are arranged in the lateral direction, the plurality of specific-color regions each having a strip shape and extending along the longitudinal direction.

4. The absorbent article according to any one of claims 1 to 3, wherein
in an unfolded and stretched state, in at least one of the longitudinal direction and the lateral direction, a total length of the specific-color region provided in one of the crotch elastic region and the peripheral region is equal to or greater than half the length of the one of the crotch elastic region and the peripheral region.

5. The absorbent article according to any one of claims 1 to 3, wherein
the first area ratio is equal to or greater than 1/2, or the second area ratio is equal to or greater than 1/2.

6. The absorbent article according to any one of claims 1 to 3, wherein
the specific-color region is not provided in the peripheral region.

7. The absorbent article according to any one of claims 1 to 3, wherein
in an unfolded and stretched state,
in the crotch elastic region, a plurality of the crotch elastic members each having a string-like shape are arranged in the lateral direction, and
a stretchable portion of the crotch elastic member has a portion that overlaps the specific-color region in the thickness direction.

8. The absorbent article according to any one of claims 1 to 3, wherein
in an unfolded and stretched state,
in the crotch elastic region, a plurality of the crotch elastic members each having a string-like shape are arranged in the lateral direction, and
in a region outside the crotch elastic region in the longitudinal direction, a non-stretchable portion of the crotch elastic member has a portion that does not overlap the specific-color region in the thickness direction.

9. The absorbent article according to any one of claims 1 to 3, wherein
the absorbent article includes:
a liquid-impermeable back sheet; and
a non-skin-side sheet provided on the non-skin side with respect to the back sheet,
the crotch elastic member is provided between the back sheet and the non-skin-side sheet,
the specific-color region is formed by coloring the back sheet, and
in an unfolded and stretched state,
in the crotch elastic region, a plurality of the crotch elastic members each having a string-like shape are arranged in the lateral direction, and
a joining portion that joins the back sheet and the non-skin-side sheet is provided between the plurality of crotch elastic members arranged in the lateral direction.

10. The absorbent article according to any one of claims 1 to 3, wherein
the absorbent article includes:
a liquid-impermeable back sheet; and
a non-skin-side sheet provided on the non-skin side with respect to the back sheet,
the crotch elastic member is provided between the back sheet and the non-skin-side sheet,
in an unfolded and stretched state,
in the crotch elastic region, a plurality of the crotch elastic members each having a string-like shape are arranged in the lateral direction,
a joining portion that joins the back sheet and the non-skin-side sheet is provided between the plurality of crotch elastic members arranged in the lateral direction, and
the joining portion extends outward beyond the crotch elastic region in the longitudinal direction.

11. The absorbent article according to any one of claims 1 to 3, wherein
the specific-color region is formed by coloring the crotch elastic member.

12. The absorbent article according to any one of claims 1 to 3, wherein
in an unfolded and stretched state,
the absorbent article includes a waist elastic region that stretches and contracts along the lateral direction at least at a longitudinal one-end portion of the absorbent article, and
when the absorbent article is viewed from the non-skin side, the waist elastic region includes a waist colored region colored along the lateral direction.

13. The absorbent article according to any one of claims 1 to 3, wherein
in an unfolded and stretched state,
the absorbent article includes side-portion elastic regions provided in two lateral side portions of an absorbent main body including the absorbent core, respectively,
the side-portion elastic regions stretching and contracting along the longitudinal direction, and
when the absorbent article is viewed from the non-skin side or a skin side, each of the side-portion elastic regions includes a side-portion colored region colored along the longitudinal direction.

14. The absorbent article according to claim 12, wherein
in an unfolded and stretched state,
the absorbent article includes side-portion elastic regions provided in two lateral side portions of an absorbent main body including the absorbent core, respectively,
the side-portion elastic regions stretching and contracting along the longitudinal direction,
when the absorbent article is viewed from the non-skin side or a skin side, each of the side-portion elastic regions includes a side-portion colored region colored along the longitudinal direction, and
the specific-color region in the crotch elastic region as viewed from the non-skin side, the waist colored region as viewed from the non-skin side, and each of the side-portion colored regions as viewed from the non-skin side or the skin side are similar in color.

15. The absorbent article according to claim 12, wherein
in an unfolded and stretched state,
the absorbent article includes side-portion elastic regions provided in two lateral side portions of an absorbent main body including the absorbent core, respectively,
the side-portion elastic regions stretching and contracting along the longitudinal direction,
when the absorbent article is viewed from the non-skin side or a skin side, each of the side-portion elastic regions includes a side-portion colored region colored along the longitudinal direction, and
a maximum lateral width of the specific-color region in the crotch elastic region is greater than a maximum longitudinal width of the waist colored region and a maximum lateral width of each of the side-portion colored regions.

16. An absorbent article having, in an unfolded and stretched state, a longitudinal direction, a lateral direction, and a thickness direction that intersect one another,
the absorbent article comprising:
an absorbent core;
a crotch elastic region delimited by a stretchable portion of a crotch elastic member; and
a peripheral region surrounding the crotch elastic region,
the crotch elastic member being provided on a non-skin side with respect to the absorbent core,
the crotch elastic member stretching and contracting along the longitudinal direction,
at least a part of the crotch elastic region being provided in a crotch portion corresponding to a central region,
the central region being obtained by dividing the absorbent article into three equal regions in the longitudinal direction,
the peripheral region being a region that, in the lateral direction, extends from a position spaced apart leftward from a left end of the crotch elastic region by a length equal to half a width of the crotch elastic region, to a position spaced apart rightward from a right end of the crotch elastic region by the length equal to half the width of the crotch elastic region, and that, in the longitudinal direction, extends from a position spaced apart toward one side from a one-side end of the crotch elastic region by a length equal to the width of the crotch elastic region, to a position spaced apart toward another side from an other-side end of the crotch elastic region by the length equal to the width of the crotch elastic region,
when the absorbent article is viewed from the non-skin side,
the absorbent article including:
a background-color region having a background color; and
a specific-color region having a specific color different from the background color,
the background-color region being a region that occupies the largest area in the crotch portion,
in at least one of the longitudinal direction and the lateral direction,
a total length of the specific-color region provided in one of the crotch elastic region and the peripheral region being equal to or greater than half a length of the one of the crotch elastic region and the peripheral region.
